# FASCICULE DE BREVET EUROPEEN

(11) **EP 2 205 602 B1**
(45) Date de publication et mention de la délivrance du brevet: **26.09.2018**
(21) Numéro de dépôt: 08805047.1
(22) Date de dépôt: 03.10.2008
(51) Int. Cl.: C07D 487/04, A61K 31/4985

(54) **IMIDAZO[1,2-A]QUINOXALINES ET DÉRIVÉS POUR LE TRAITEMENT DES CANCERS**
IMIDAZO[1,2-A]CHINOXALINE UND DERIVATE DAVON ZUR BEHANDLUNG VON KREBS
IMIDAZO[1,2-A]QUINOXALINES AND DERIVATIVES THEREOF FOR TREATING CANCERS

(30) Priorité: 03.10.2007 FR 0706928
(43) Date de publication de la demande: 14.07.2010
(73) Titulaire: Université de Montpellier, 34090 Montpellier (FR); American University Of Beirut, Beyrouth 11072020 (LB); Centre Regional De Lutte Contre Le Cancer De Montpellier, 34298 Montpellier (FR)
(72) Inventeur: DELEUZE-MASQUEFA, Carine, F-34430 Saint-jean De Vedas (FR); MOARBESS, Georges, Zghorta 1304 (LB); BONNET, Pierre-Antoine, F-34080 Montpellier (FR); PINGUET, Frédéric, F-34570 Montarnaud (FR); BAZARBACHI, Ali, Beirut (LB); BRESSOLLE, Françoise, F-34000 Montpellier (FR)
(74) Mandataire: Regimbeau
(86) Numéro de dépôt international: PCT/EP2008/063290
(87) Numéro de publication internationale: WO 2009/043934

(56) Documents cités:
- WO-A-93/04066
- WO-A-2007/087250
- WO-A-2007/109813
- MORJARIA S ET AL: "Impairment of TNF-.alpha. production and action by imidazo(1,2-.alpha.) quinoxalines, a derivative family which displays potential anti-inflammatoryproperties" INTERNATIONAL JOURNAL OF IMMUNOPATHOLOGY AND PHARMACOLOGY, BIOMEDICAL RESEARCH PRESS, XX, vol. 19, no. 3, 1 janvier 2006 (2006-01-01), pages 525-538, XP009086226 ISSN: 0394-6320 cité dans la demande
- DELEUZE-MASQUEFA, CARINE ET AL: "Design and synthesis of novel imidazo[1,2-a]quinoxalines as PDE4 inhibitors" BIOORGANIC & MEDICINAL CHEMISTRY, vol. 12, no. 5, 2004, pages 1129-1139, XP002488139 cité dans la demande
- COLOTTA, V. ET AL: "Synthesis of some tricyclic heteroaromatic systems and their A1 and A2a adenosine binding activity" EUROPEAN JOURNAL OF MEDICINAL CHEMISTRY , 30(2), 133-9, vol. 30, no. 2, 1995, pages 133-139, XP002488140
- CATARZI, DANIELA ET AL: "Structure-Activity Relationships of 1,2,4-Triazolo[1,5- a]quinoxalines and Their 1-Deaza Analogs Imidazo[1,2-a]quinoxalines at the Benzodiazepine Receptor" JOURNAL OF MEDICINAL CHEMISTRY, vol. 37, no. 18, 1994, pages 2846-2850, XP002488141
- ZURBONSEN K ET AL: "APOPTOTIC EFFECTS OF IMIDAZO not 1,2-ALPHA 3/4 PYRAZINE DERIVATIVES IN THE HUMAN DAMI CELL LINE" EUROPEAN JOURNAL OF PHARMACOLOGY, vol. 320, 1997, pages 215-221, XP002924561 ISSN: 0014-2999
- CORONA, PAOLA ET AL: "4-Substituted anilino imidazo[1,2-a] and triazolo[4,3-a]quinoxalines. Synthesis and evaluation of in vitro biological activity" EUROPEAN JOURNAL OF MEDICINAL CHEMISTRY, vol. 41, no. 9, 11 septembre 2006 (2006-09-11), pages 1102-1107, XP005651184 EDITIONS SCIENTIFIQUE ELSEVIER, PARIS, FR ISSN: 0223-5234
- ZURBONSEN K ET AL: "Antiproliferative effects of Imidazo[1,2-a]pyrazine derivatives on the Dami cell Line" BIOCHEMICAL PHARMACOLOGY, vol. 54, 1997, pages 365-371, XP002267821 ISSN: 0006-2952

## Description

La présente invention concerne des composés imidazo[1,2-*a*]quinoxaline pour le traitement des cancers ainsi que les compositions pharmaceutiques comprenant ces composés et leurs utilisations en thérapie. L'invention a également pour objet l'utilisation de composés dérivés d'imidazo[1,2-*a*]quinoxaline pour la préparation de médicaments pour le traitement des cancers et en particulier pour le traitement des mélanomes et des lymphomes à cellules T.

Face à la faible efficacité d'une grande majorité d'anticancéreux classiques dans le traitement de cancers tels que la prostate, le colon, le sein, le mélanome, et encore le lymphome, les recherches s'orientent vers de nouvelles stratégies thérapeutiques. En effet, réussir à contourner les problèmes de résistances et de métastases, rencontrés de plus en plus souvent dans ce type de maladie, représente un défi important pour la recherche.

### Le mélanome

Responsable de plusieurs milliers de décès chaque année en France, le cancer de la peau est un des cancers les plus redoutables. Sa fréquence augmente très fortement avec l'âge, avec la diminution de l'ozone et avec une forte exposition au soleil. Compte tenu de l'augmentation croissante de l'espérance de vie et de l'augmentation de sa fréquence, le cancer de la peau sera bientôt l'un des problèmes de santé publique les plus importants. Le mélanome, qui ressemble à un simple grain de beauté, est le plus grave des cancers cutanés. Un mélanome peut être bénin (naevus) ou malin (mélanome malin ou mélanoblastome). Les mélanomes malins ne représentent certes que 10% des cancers de la peau, mais ils sont mortels s'ils ne sont pas traités. C'est l'un des cancers les plus agressifs qui soient, mais le traitement à une phase initiale permet de guérir le patient. Un mélanome malin non traité envahit rapidement la peau et progresse vers le reste de l'organisme particulièrement le foie, les poumons, les os et le cerveau, entraînant la mort du patient en quelques mois.

### La leucémie T de l'adulte liée au virus HTLV-1

Le virus *HTLV1* (Human T cell leukemia/lymphoma virus type 1) est le premier rétrovirus oncogène découvert chez l'homme en 1980. La leucémie T de l'adulte: "Adult T-cell leukemia" ou ATL est une prolifération lymphoïde maligne, portant sur les lymphocytes T généralement CD4+. Ces lymphocytes T expriment sur leur surface les marqueurs lymphocytaires T CD2, CD3, CD4, CD5, CD45RO mais n'expriment pas le marqueur CD7 et rarement le CD8. Les cellules d'ATL sont caractérisées par la présence du provirus HTLV-I intégré de manière monoclonale, rarement oligoclonale.

La diversité de la présentation clinique et l'évolution très différente selon le mode de présentation ont justifié leur classification en quatre formes cliniques qui peuvent se succéder l'une à l'autre :
- La forme aiguë de l'ATL (>5%). Cette pathologie, qui survient chez l'adulte, est de très sombre pronostic avec une résistance ou une rechute précoce même après des polychimiothérapies intensives. La médiane de survie est de l'ordre de 6 mois.
- La forme chronique de l'ATL a une évolution plus lente, avec des signes cliniques peu sévères. Elle présente une lymphocytose avec un nombre élevé de cellules leucémiques (>5).
- Une forme moins sévère: l'ATL subaiguë dite smouldering (rampante), caractérisée par une évolution progressive sur une longue période, avec une présence de quelques cellules leucémiques dans le sang circulant (1-5%).
- Une forme lymphomateuse est caractérisée par un faible taux de cellules leucémiques dans le sang circulant (< 1%). Comme la forme aiguë, elle présente un pronostic très sévère.

### Les lymphomes périphériques à cellules T

Ils représentent environ 15% de l'ensemble des lymphomes non-Hodgkiniens. Ces lymphomes T ont un pronostic relativement sévère, avec un taux de rémission complète nettement inférieur à celui des lymphomes B, et un taux de rechute nettement supérieur. De plus, ces lymphomes T n'ont pas bénéficié des progrès thérapeutiques dans les lymphomes B dues à l'utilisation du rituximab, anticorps monoclonal anti CD20.

De grands espoirs ont été placés dans l'immunothérapie qui contrairement aux autres thérapies, permet de traiter l'organisme dans sa globalité et peut éliminer les cellules tumorales disséminées dans l'ensemble du corps. Parmi les différentes approches d'immunothérapies, la découverte de l'imiquimod (Aldara®), premier anticancéreux immunomodulateur, efficace notamment contre certains cancers de la peau tel que le mélanome, a permis de faire un pas en avant dans cette nouvelle voie. L'imiquimod est une molécule tricyclique azotée, de la famille des imidazoquinolines (WO 2006/070408, US 4,689,338). Elle est tout d'abord connue pour son activité antivirale contre certains virus tels que l'herpès simplex II, le sendaï virus et le papilloma virus. Les dernières publications parues sur cette molécule mettent aussi en évidence une activité antitumorale immunomodulatrice importante sur les cancers de la peau, tels que les carcinomes basocellulaires, les kératoses actiniques et les mélanomes. Des études plus récentes ont montré également une efficacité contre les métastases cutanées et les tumeurs vasculaires. L'imiquimod est ainsi le premier d'une nouvelle classe de médicaments anticancéreux appelés modificateurs de la réponse immunitaire innée et acquise dont le mécanisme d'action diffère de tous les anticancéreux connus comme les moutardes à l'azote, les nitroso-urées, les agents alkylants, les organo-platines, etc..

Deleuze-Masquéfa et al. (Bioorganic & Medicinal Chemistry 12 : 1129-1139, 2004) ont décrit des dérivés imidazo[1,2-*a*]quinoxalines comme inhibiteurs des PDE4 (phosphodiesterases 4). Contrairement à l'imiquimod, ces molécules inhibent la production et les effets du TNF-α *in vitro* et elles semblent donc avoir un mode d'action différent de l'imiquimod. Ces dérivés pourraient avoir un intérêt pour leurs propriétés anti-inflammatoires (Mojaria et al. International Journal of Immunopathology and Pharmacology, Vol. 19, no. 2, 77-90, 2006). Une activité anticancéreuse, et à fortiori une activité sur les mélanomes ou les lymphomes, n'est ni décrite ni suggérée dans ces documents.

Bonnard *et al.* (RICT 2005, Paris) ont décrit la synthèse et l'évaluation pour leur activité antitumorale de dérivés imidazo[1,2-*a*]quinoxalines. La structure des composés testés n'est cependant pas décrite.

US 2003/0022898 décrit des dérivés ayant également une activité anti-inflammatoire dont un composé 4-(2'-aminoéthyl)-amino-1,8-diméthylimidazo(1,2-*a*)quinoxaline. Ce composé est également décrit comme ayant une activité anti-mélanome dans le document US 2006/0025419.

Colotta et al. (Eur. J. Med. Chem, 30,133-139, 1995) décrit différents composés dont des imidazo[1,2-*a*]quinoxalines. Des triazoloquinoxalines sont décrites comme se liant au récepteur adénosine. Des applications particulières des imidazo[1,2-*a*]quinoxalines ne sont pas décrites.

Catarzi et al. (J. Med. Chem., 37, 2846-2850, 1994) décrit des triazoloquinoxalines ainsi que des imidazoquinoxalines se liant au récepteur de la benzodiazépine. Les molécules se liant au récepteur de la benzodiazépine sont en général reconnues pour leurs activités anxiolytiques. Ce document ne décrit pas d'applications dans le cancer.

WO 93/04066 décrit des composés imidazoquinoxalinols se liant spécifiquement au récepteurs GABAa. Seules des utilisations thérapeutiques comme sédatifs, anxiolytiques, anticonvulsifs, etc. sont envisagées. Des applications dans d'autres domaines thérapeutiques et notamment pour le traitement des cancers ne sont ni décrites ni suggérées par ce document.

Zurbonsen et al. (European Journal of Pharmacology, 320, 215-221, 1997 et Biochemical Pharmacology, 54, 365-371, 1997) décrivent des dérivés d'imidazo[1,2-*a*]pyrazines ayant une activité inhibitrice sur les phosphodiéstérases et capables d'induire l'apoptose d'une lignée cellulaire leucémique. Il est cependant à noter que tous les inhibiteurs des phosphodiestérases ne présentent pas une activité anticancéreuse. En outre, les composés de la présente invention se distinguent par une activité élevée démontrée dans des tests *in vitro* et *in vivo* aussi bien sur lymphome que sur le mélanome.

WO 2007/109813 concerne des imidazoquinoxalines ayant une activité immunomodulatrice. Seules des applications dans le domaine des adjuvants et des vaccins sont décrites. Ce document envisage des applications potentielles dans de nombreux domaines thérapeutiques dont le cancer mais aucune donnée *in vitro* ou *in vivo* vient confirmer une quelconque activité anticancéreuse.

WO 2007/087250 décrit des inhibiteurs de la 5-LO (lipoxygénase). Des applications dans le domaine du cancer ne sont pas décrites.

Corona et al. (European Journal of Medicinal Chemistry 41(2006) 1102-1107) décrit des composés 4-anilinoimidazo[1,2-*a*]quinoxalines et 4-anilinotriazolo[4,3-*a*]quinoxalines ayant des propriétés antiprolifératives sur des cellules MT-4.

Pour remédier aux inconvénients de l'état de la technique, la présente invention propose des composés dérivés d'imidazo[1,2-*a*]quinoxalines pour le traitement des cancers et plus particulièrement les mélanomes et les lymphomes à cellules T. Ces composés peuvent être utilisés pour la préparation de médicaments pour le traitement des cancers.

Avantageusement, les composés de la présente invention ont une efficacité améliorée ainsi qu'une faible toxicité. Les composés de la présente invention ont par exemple une efficacité améliorée par rapport à l'imiquimod.

En outre, les composés de la présente invention ont démontré une activité sur le mélanome mais aussi sur le lymphome dans des tests *in vitro* et *in vivo.*

Avantageusement, les composés de la présente invention n'ont pas d'activité pro-inflammatoire contrairement à d'autres molécules utilisés dans le traitement des cancers tel que l'imiquimod.

La présente invention concerne des composés, compositions et produits pour le traitement des cancers tels que définis dans les revendications 1 à 10.

### DESCRIPTION

La description concerne des composés de formule générale (I) : 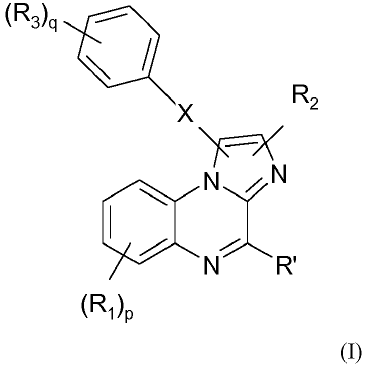 dans laquelle
R₁, R₂, R₃ et R' représentent indépendamment un atome d'hydrogène, un halogène ou un groupe choisi parmi les groupes hydroxy, alkyle, alkényle, cycloalkyle, thioalkyle, alcoxy, amino, alkylamino, dialkylamino, acyle, aryle, aralkyle ou un hétérocycle saturé ou non, éventuellement substitués par un ou plusieurs substituants, et les radicaux cyano, nitroso, nitro, -CF₃, - (CH₂)ₙNR₄R₅, -(CH₂)ₙCOR₄, -(CH₂)ₙCO-NR₄R₅, -(CH₂)ₙSO₂-NR₄R₅, -(CH₂)ₙCO₂R₄, -NH-(CH₂)ₙNR₄R₅
n compris entre 0 et 4,
p compris entre 1 et 4,
q compris entre 1 et 5,
X représente (CH₂)ₘ, (CH₂)ₘO(CH₂)_{m'}, (CH₂)ₘNH(CH₂)_{m'},
m compris entre 0 et 4,
m' compris entre 0 et 4,
R₄ et R₅ représentent indépendamment un atome d'hydrogène ou groupe choisi parmi les radicaux alkyle en C₁-C₄ linéaire ou ramifié, alkényle en C₁-C₄ linéaire ou ramifié, cycloalkyle en C₃-C₇, acyle, aryle, aralkyle ou un hétérocycle, éventuellement substitués par un ou plusieurs substituants, et ses sels physiologiquement acceptables.

Préférentiellement, R₁, R₂, R₃, et R' représentent indépendamment un atome d'hydrogène, un halogène ou un groupe choisi parmi les groupes hydroxyle, alkyle de C₁ à C₄, alkényle de C₂ à C₄, thioalkyle de C₁ à C₄, alcoxy de C₁ en C₄, amino, alkylamino de C₁ à C₄, dialkylamino de C₁ à C₄,acyle de C₁ à C₄, aryle de C₁ à C₄, aralkyle de C₁ à C₄, éventuellement substitués par un ou plusieurs substituants, et les radicaux cyano, nitroso, nitro, -CF₃, (CH₂)ₙNR₄R₅, -(CH₂)ₙCOR₄, - (CH₂)ₙCO-NR₄R₅, -(CH₂)ₙSO₂-NR₄R₅, -(CH₂)ₙCO₂R₄, -NH-(CH₂)ₙNR₄R₅,
n compris entre 0 et 4,
p compris entre 1 et 4
q compris entre 1 et 5.

R₄ et R₅ représentent indépendamment un atome d'hydrogène ou groupe choisi parmi les radicaux alkyle en C₁-C₄ linéaire ou ramifié alkényle en C₁-C₄ linéaire ou ramifié, cycloalkyle en C₃-C₇.

De préférence, R₁, R₂ et R₃ représentent indépendamment un atome d'hydrogène, un halogène ou un groupe choisi parmi les groupes méthyle, éthyle, propyle, isopropyle, butyle, isobutyle, tertiobutyle, et -(CH₂)_{n"}-(CH=CH)-(CH₂)_{n'''}-CH₃ avec n" et n''' compris indépendamment entre 0 et 4, COOR₄, NR₄R₅ et OR₄, R₄ et R₅ sont définis ci-dessus.
R' représente indépendamment un atome d'hydrogène, un halogène ou un groupe choisi parmi les groupes hydroxy, alcoxy, amino, alkylamino, dialkylamino, un hétérocycle saturé ou non et NH-(CH₂)ₙNR₄R₅.

Préférentiellement, R₁ est l'hydrogène.

Préférentiellement, R₂ est l'hydrogène.

Préférentiellement, R₃ est choisi parmi H, Cl, Br, F, hydroxy, méthyl, méthoxy, éthoxy, CF₃, CN, COOH, COOCH₃, COOCH₂CH₃, COONH₂, CHO, NO₂ et C₄H₃O. Plus préférentiellement, R₃ est choisi parmi H, hydroxy, méthoxy, éthoxy, Br, CF₃, Cl et COOH.

De préférence, X est choisi parmi O, NH, CH₂, (CH₂)₂, O(CH₂)_{m'}, et NH(CH₂)_{m'}, avec m' défini ci-dessus. Préférentiellement, X est (CH₂)₂.

De préférence, R' est un groupe chloro, méthoxy, amino, méthylamino, diméthylamino, éthylamino, diéthylamino, aminométhylamine ou aminoéthylamine. Préférentiellement, R' est - NH-CH₃, -NH₂ ou -NH-(CH₂)₂-NH₂.

De préférence, q=1 ou 2. Préférentiellement, q=1.

Préférentiellement, q= 1 et R₃ est en position 3 ou 4 sur le groupement phényle.

Les substituants sont préférentiellement choisis parmi les halogènes, les groupes hydroxyles, cyano, nitroso, nitro, -CF3, alkyle, alkényle, cycloalkyle, thioalkyle, alcoxy, amino, alkylamino, acyle, aryle, aralkyle ou un hétérocycle saturé ou non.

Dans un premier mode de réalisation, la description concerne des composés répondant à la formule générale (II) : 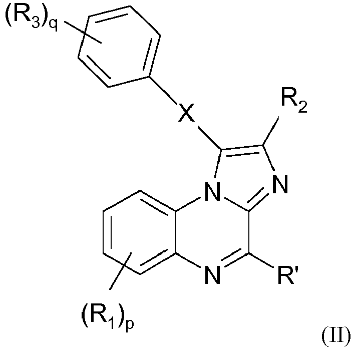 dans laquelle R₁, R₂, R₃, R', X, p et q sont définis ci-dessus.

Dans un deuxième mode de réalisation, la description concerne des composés répondant à la formule générale (III) : 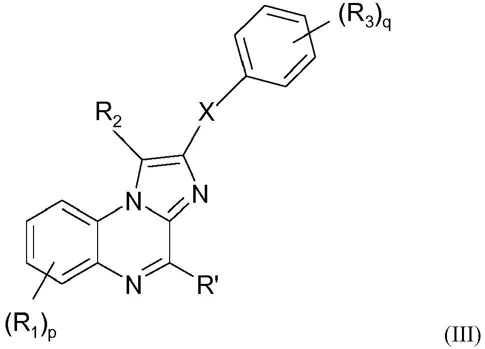 dans laquelle
R₁, R₂, R₃, R', X, p et q sont définis ci-dessus.

Préférentiellement, la description concerne des composés choisis parmi les composés suivants : *N*-Méthyl-2-(2-phényléthyl)imidazo[1,2-*a*]quinoxalin-4-amine, *N*,*N*-Diméthyl-2-(2-phényléthyl)imidazo[1,2-*a*]quinoxalin-4-amine, *N*-(2-aminoéthyl)-2-(2-phényléthyl)imidazo[1,2-*a*]quinoxalin-4-amine, 2-(2-phényléthyl)imidazo[1,2-*a*]quinoxalin-4-amine, 4-Méthoxy-2-(2-phényléthyl)imidazo[1,2-*a*]quinoxaline, 4-Chloro-2-(2-phényléthyl)imidazo[1,2-*a*]quinoxaline et leurs sels physiologiquement acceptables.

De préférence, la description concerne des composés répondant à la formule générale (IV) : 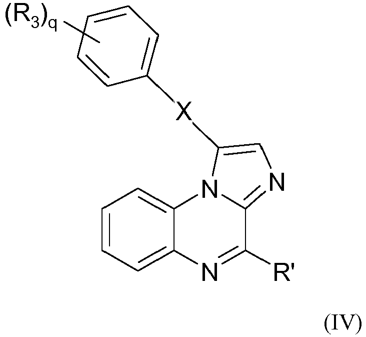 dans laquelle R₃, R', X et q sont définis ci-dessus.

De préférence, les composés selon la description sont choisis parmi les composés suivants : *N*-Méthyl-1-(2-phényléthyl)imidazo[1,2-*a*]quinoxalin-4-amine, *N*,*N*-1-(2-phényléthyl)imidazo[1,2-*a*]quinoxalin-4-amine, *N*-(2-aminoéthyl)-1-(2-phényléthyl)imidazo[1,2-*a*]quinoxalin-4-amine, 1-(2-phényléthyl)imidazo[1,2-*a*]quinoxalin-4-amine, 4-Méthoxy-1-(2-phényléthyl)imidazo[1,2-*a*]quinoxaline, 4-Chloro-1-(2-phényléthyl)imidazo[1,2-*a*]quinoxaline, 1-(2-phényléthyl)imidazo[1,2-*a*]quinoxaline-4(5H)-one, 1 -(2-phényléthyl)-4-pyrrolidin-1 - yl]imidazo[1,2-*a*]quinoxaline, 1-(2-phényléthyl)-4-piperidin-1-yl]imidazo[1,2-*a*]quinoxaline, N-phenyl-1-(2-phényléthyl)imidazo [1,2-*a*]quinoxalin-4-amine, *N*,1-bis(2-phényléthyl)imidazo [1,2-*a*]quinoxalin-4-amine, *t*-butyl-4-[1-(2-phenylethyl)imidazo[1,2-*a*]quinoxalin-4-yl]piperazine-1-carboxylate, [1-(2-phényléthyl)-4-piperazin-1-yl]imidazo[1,2-*a*]quinoxaline et leurs sels physiologiquement acceptables .

Dans un mode de réalisation avantageux de la description, les composés répondent à la formule générale (V) : 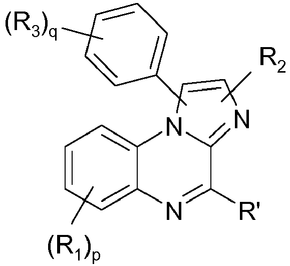 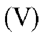 dans laquelle R₁, R₂, R₃, R', p et q sont définis ci-dessus.
Dans un autre mode de réalisation de la description, les composés répondent à la formule générale (VI) : 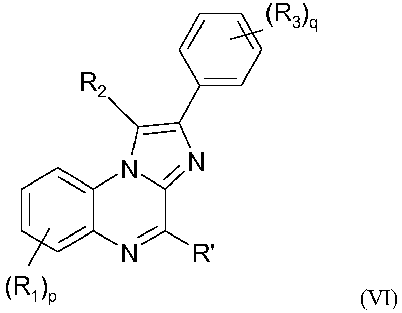 dans laquelle R₁, R₂, R₃, R', p et q sont définis ci-dessus.

De préférence, les composés répondent à la formule générale (VII) : 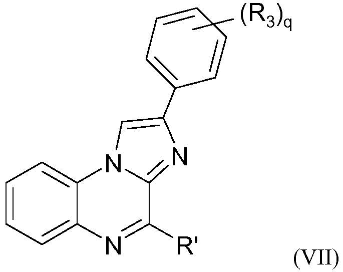 dans laquelle R₃, R' et q sont définis ci-dessus.

De préférence, les composés sont choisis parmi les composés suivants : *N*-(2-aminoéthyl)-2-(2-hydroxyphényl)imidazo[1,2-*a*]quinoxalin-4-amine, *N*-(2-aminoéthyl)-2-(3-hydroxyphényl)imidazo[1,2-*a*]quinoxalin-4-amine, *N*-(2-aminoéthyl)-2-(4-hydroxyphényl)imidazo[1,2-*a*]quinoxalin-4-amine, *N*-(2-aminoéthyl)-2-(2,4-dihydroxyphényl)imidazo[1,2-*a*]quinoxalin-4-amine, *N*-(2-aminoéthyl)-2-(2,3-dihydroxyphényl)imidazo[1,2-*a*]quinoxalin-4-amine, *N*-(2-aminoéthyl)-2-(2-méthoxyphényl)imidazo[1,2-*a*]quinoxalin-4-amine, *N*-(2-aminoéthyl)-2-(3-méthoxyphényl)imidazo[1,2-*a*]quinoxalin-4-amine, *N*-(2-aminoéthyl)-2-(4-méthoxyphényl)imidazo[1,2-*a*]quinoxalin-4-amine, *N*-(2-aminoéthyl)-2-(2,4-diméthoxyphényl)imidazo[1,2-*a*]quinoxalin-4-amine, *N*-(2-aminoéthyl)-2-(2,3-diméthoxyphényl)imidazo[1,2-*a*]quinoxalin-4-amine, *N*-(2-aminoéthyl)-2-(3-éthoxyphényl)imidazo[1,2-*a*]quinoxalin-4-amine, *N*-(2-aminoéthyl)-2-(4-éthoxyphényl)imidazo[1,2-*a*]quinoxalin-4-amine, *N*-(2-aminoéthyl)-2-(3-bromophényl)imidazo[1,2-*a*]quinoxalin-4-amine, *N*-(2-aminoéthyl)-2-(4-bromophényl)imidazo[1,2-*a*]quinoxalin-4-amine, *N*-(2-aminoéthyl)-2-(3-(trifluorométhyl)phényl))imidazo[1,2-*a*]quinoxalin-4-amine, *N*-(2-aminoéthyl)-2-(4-(trifluorométhyl)phényl))imidazo[1,2-*a*]quinoxalin-4-amine, *N*-(2-aminoéthyl)-2-(3-chlorophényl)imidazo[1,2-*a*]quinoxalin-4-amine, *N*-(2-aminoéthyl)-2-(4-chlorophényl)imidazo[1,2-*a*]quinoxalin-4-amine, *N*-(2-aminoéthyl)-2-(3-carboxyphényl)imidazo[1,2-*a*]quinoxalin-4-amine, *N*-(2-aminoéthyl)-2-(4-carboxyphényl)imidazo[1,2-a]quinoxalin-4-amine, *N*-(2-aminoéthyl)-2-(3-fluorophényl)imidazo[1,2-*a*]quinoxalin-4-amine, *N*-(2-aminoéthyl)-2-(4-fluorophényl)imidazo[1,2-*a*]quinoxalin-4-amine, *N*-(2-aminoéthyl)-2-(3-cyanophényl)imidazo[1,2-*a*]quinoxalin-4-amine, *N-*(2-aminoéthyl)-2-(4-cyanophényl)imidazo[1,2-*a*]quinoxalin-4-amine, *N*-(2-aminoéthyl)-2-(3-nitrophényl)imidazo[1,2-*a*]quinoxalin-4-amine, *N*-(2-aminoéthyl)-2-(4-nitrophényl)imidazo[1,2-*a*]quinoxalin-4-amine, *N*-(2-aminoéthyl)-2-furanimidazo[1,2-*a*]quinoxalin-4-amine,N-Méthyl-2-phénylimidazo[1,2-*a*]quinoxalin-4-amine, *N*-méthyl-2-(2-hydroxyphényl)imidazo[1,2-*a*]quinoxalin-4-amine, *N-*méthyl-2-(3-hydroxyphényl)imidazo[1,2-*a*]quinoxalin-4-amine, *N-*méthyl-2-(4-hydroxyphényl)imidazo[1,2-*a*]quinoxalin-4-amine, N-Méthyl-2-(2,4-dihydroxyphényl)imidazo[1,2-*a*]quinoxalin-4-amine, N-Méthyl-2-(2,3-dihydroxyphényl)imidazo[1,2-*a*]quinoxalin-4-amine, N-Méthyl-2-(2-méthoxyphényl)imidazo[1,2-*a*]quinoxalin-4-amine, N-Méthyl-2-(3-méthoxyphényl)imidazo[1,2-*a*]quinoxalin-4-amine, N-Méthyl-2-(4-méthoxyphényl)imidazo[1,2-*a*]quinoxalin-4-amine, N-Méthyl-2-(2,4-diméthoxyphényl)imidazo[1,2-*a*]quinoxalin-4-amine, N-Méthyl-2-(2,3-diméthoxyphényl)imidazo[1,2-*a*]quinoxalin-4-amine, 2-phénylimidazo[1,2-*a*]quinoxalin-4-amine, N-Méthyl-2-(3-éthoxyphényl)imidazo[1,2-*a*]quinoxalin-4-amine, N-Méthyl-2-(4-éthoxyphényl)imidazo[1,2-*a*]quinoxalin-4-amine, *N*-méthyl-2-(3-bromophényl)imidazo[1,2-*a*]quinoxalin-4-amine, *N*-méthyl-2-(4-bromophényl)imidazo[1,2-*a*]quinoxalin-4-amine, *N*-méthyl-2-(3-(trifluorométhyl)phényl))-imidazo[1,2-*a*]quinoxalin-4-amine, *N*-méthyl-2-(4-(trifluorométhyl)-phényl))imidazo[1,2-*a*]quinoxalin-4-amine, *N*-méthyl-2-(3-chlorophényl)imidazo[1,2-*a*]quinoxalin-4-amine, *N*-méthyl-2-(4-chlorophényl)-imidazo[1,2-*a*]quinoxalin-4-amine, *N*-méthyl-2-(3-carboxyphényl)imidazo[1,2-*a*]quinoxalin-4-amine, *N-*méthyl-2-(4-carboxyphényl)imidazo[1,2-*a*]quinoxalin-4-amine, *N*-méthyl-2-(3-fluorophényl)imidazo[1,2-*a*]quinoxalin-4-amine, *N*-méthyl-2-(4-fluorophényl)imidazo[1,2-*a*]quinoxalin-4-amine, *N*-méthyl-2-(3-cyanophényl)imidazo[1,2-*a*]quinoxalin-4-amine, *N*-méthyl-2-(4-cyanophényl)imidazo[1,2-*a*]quinoxalin-4-amine, *N*-méthyl-2-(3-nitrophényl)imidazo[1,2-*a*]quinoxalin-4-amine, *N*-méthyl-2-(4-nitrophényl)imidazo[1,2-*a*]quinoxalin-4-amine, *N*-méthyl-2-furanimidazo[1,2-*a*]quinoxalin-4-amine, 2-(2-hydroxyphényl)imidazo[1,2-*a*]quinoxaline, 2-(3-hydroxyphényl)imidazo[1,2-*a*]quinoxaline, 2-(4-hydroxyphényl)imidazo[1,2-*a*]quinoxaline, 2-(2,4-dihydroxyphényl)imidazo[1,2-*a*]quinoxalin-4-amine, 2-(2,3-dihydroxyphényl)imidazo[1,2-*a*]quinoxalin-4-amine, 2-(2-méthoxyphényl)imidazo[1,2-*a*]quinoxalin-4-amine, 2-(3-méthoxyphényl)imidazo[1,2-*a*]quinoxalin-4-amine, 2-(4-méthoxyphényl)imidazo[1,2-*a*]quinoxalin-4-amine, 2-(2,4-diméthoxyphényl)imidazo[1,2-*a*]quinoxalin-4-amine, 2-(2,3-diméthoxyphényl)imidazo[1,2-*a*]quinoxalin-4-amine, 2-(3-éthoxyphényl)imidazo[1,2-*a*]quinoxalin-4-amine, 2-(4-éthoxyphényl)imidazo[1,2-*a*]quinoxalin-4-amine, 2-(3-bromophényl)imidazo[1,2-*a*]quinoxalin-4-amine, 2-(4-bromophényl)imidazo[1,2-*a*]quinoxalin-4-amine, 2-(3-(trifluorométhyl)phényl))imidazo[1,2-*a*]quinoxalin-4-amine, 2-(4-(trifluorométhyl)phényl))imidazo[1,2-*a*]quinoxalin-4-amine, 2-(3-chlorophényl)imidazo[1,2-*a*]quinoxalin-4-amine, 2-(4-chlorophényl)imidazo[1,2-*a*]quinoxalin-4-amine, 2-(3-carboxyphényl)imidazo[1,2-*a*]quinoxalin-4-amine, 2-(4-carboxyphényl)imidazo[1,2-*a*]quinoxalin-4-amine, 2-(3-fluorophényl)imidazo[1,2-*a*]quinoxalin-4-amine, 2-(4-fluorophényl)imidazo[1,2-*a*]quinoxalin-4-amine, 2-(3-cyanophényl)imidazo[1,2-*a*]quinoxalin-4-amine, 2-(4-cyanophényl)imidazo[1,2-*a*]quinoxalin-4-amine, 2-(3-nitrophényl)imidazo[1,2-*a*]quinoxalin-4-amine, 2-(4-nitrophényl)imidazo[1,2-*a*]quinoxalin-4-amine, 2-furanimidazo[1,2-*a*]quinoxalin-4-amine,4-Chloro-2-phénylimidazo[1,2-*a*]quinoxaline, 4-Chloro-2-(2-hydroxyphényl)imidazo[1,2-*a*]quinoxaline, 4-Chloro-2-(3-hydroxyphényl)imidazo[1,2-*a*]quinoxaline, 4-Chloro-2-(4-hydroxyphényl)imidazo[1,2-*a*]quinoxaline, 4-Chloro-2-(2-méthoxyphényl)imidazo[1,2-*a*]quinoxaline, 4-Chloro-2-(3-méthoxyphényl)imidazo[1,2-*a*]quinoxaline, 4-Chloro-2-(4-méthoxyphényl)imidazo[1,2-*a*]quinoxaline, 4-Chloro-2-(2,4-diméthoxyphényl)-imidazo[1,2-*a*]quinoxaline, 4-Chloro-2-(2,3-diméthoxyphényl)imidazo[1,2-*a*]quinoxaline, 4-Méthoxy-2-phénylimidazo[1,2-*a*]quinoxaline, 4-Méthoxy-2-(2-hydroxyphényl)-imidazo[1,2-*a*]quinoxaline, 4-Méthoxy-2-(3-hydroxyphényl)imidazo[1,2-*a*]quinoxaline, 4-Méthoxy-2-(4-hydroxyphényl)imidazo[1,2-*a*]quinoxaline, 4-Méthoxy-2-(2-méthoxyphényl)imidazo[1,2-*a*]quinoxaline, 4-Méthoxy-2-(3-méthoxyphényl)imidazo[1,2-*a*]quinoxaline, 4-Méthoxy-2-(4-méthoxyphényl)imidazo[1,2-*a*]quinoxaline, 4-Méthoxy-2-(2,4-diméthoxyphényl)imidazo[1,2-*a*]quinoxaline, 4-Méthoxy-2-(2,3-diméthoxyphényl)imidazo[1,2-*a*]quinoxaline, et leurs sels physiologiquement acceptables.

Dans un mode de réalisation de la description, les composés répondent à la formule générale (VIII) : 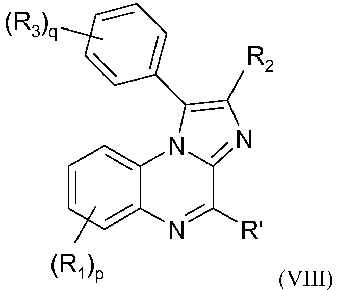 dans laquelle R₁, R₂, R₃, R', p et q sont définis ci-dessus.

De préférence, les composés de l'invention répondent à la formule générale (IX) : 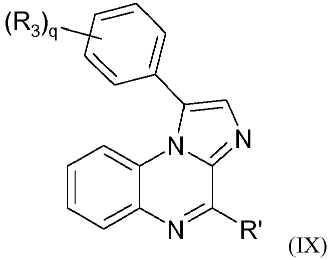 dans laquelle R₃, R' et q sont définis ci-dessus.

De préférence, les composés sont choisis parmi les composés suivants : N-Méthyl-1-phénylimidazo[1,2-*a*]quinoxalin-4-amine, *N*-méthyl-1-(2-hydroxyphényl)imidazo[1,2-*a*]quinoxalin-4-amine, *N*-méthyl-1-(3-hydroxyphényl)imidazo[1,2-*a*]quinoxalin-4-amine, *N-*méthyl-1 -(4-hydroxyphényl)imidazo [1,2-*a*]quinoxalin-4-amine, N-Méthyl-1-(2,4-dihydroxyphényl)imidazo[1,2-*a*]quinoxalin-4-amine, N-Méthyl-1-(2,3-dihydroxyphényl)imidazo[1,2-*a*]quinoxalin-4-amine, N-Méthyl-1-(2-méthoxyphényl)imidazo[1,2-*a*]quinoxalin-4-amine, N-Méthyl-1-(3-méthoxyphényl)imidazo[1,2-*a*]quinoxalin-4-amine, N-Méthyl-1-(4-méthoxyphényl)imidazo[1,2-*a*]quinoxalin-4-amine, *N*-Méthyl-1-(2,4-diméthoxyphényl)imidazo[1,2-*a*]quinoxalin-4-amine, N-Méthyl-1-(2,3-diméthoxyphényl)imidazo[1,2-*a*]quinoxalin-4-amine, N-Méthyl-1-(3-éthoxyphényl)imidazo[1,2-*a*]quinoxalin-4-amine, N-Méthyl-1-(4-éthoxyphényl)imidazo[1,2-*a*]quinoxalin-4-amine, *N*-méthyl-1-(3-bromophényl)imidazo[1,2-*a*]quinoxalin-4-amine, *N*-méthyl-1-(4-bromophényl)imidazo[1,2-*a*]quinoxalin-4-amine, *N*-méthyl1-(3-(trifluorométhyl)phényl))imidazo[1,2-*a*]quinoxalin-4-amine, *N*-méthyl-1-(4-(trifluorométhyl)phényl))imidazo[1,2-*a*]quinoxalin-4-amine, *N*-méthyl-1-(3-chlorophényl)imidazo[1,2-*a*]quinoxalin-4-amine, *N*-méthyl-1-(4-chlorophényl)imidazo[1,2-*a*]quinoxalin-4-amine, *N*-méthyl-1-(3-carboxyphényl)imidazo[1,2-*a*]quinoxalin-4-amine, *N-*méthyl-1 -(4-carboxyphényl)imidazo [1,2-*a*]quinoxalin-4-amine, *N*-méthyl-1-(3-fluorophényl)imidazo[1,2-*a*]quinoxalin-4-amine, *N*-méthyl-1-(4-fluorophényl)imidazo[1,2-*a*]quinoxalin-4-amine, *N*-méthyl-1-(3-cyanophényl)imidazo[1,2-*a*]quinoxalin-4-amine, *N*-méthyl-1-(4-cyanophényl)imidazo[1,2-*a*]quinoxalin-4-amine, *N*-méthyl-1-(3-nitrophényl)imidazo[1,2-*a*]quinoxalin-4-amine, *N*-méthyl-1-(4-nitrophényl)imidazo[1,2-*a*]quinoxalin-4-amine, *N*-méthyl-1-furanimidazo[1,2-*a*]quinoxalin-4-amine, *N*-(2-aminoéthyl)-1-(2-hydroxyphényl)imidazo[1,2-*a*]quinoxalin-4-amine, *N*-(2-aminoéthyl)-1-(3-hydroxyphényl)imidazo[1,2-*a*]quinoxalin-4-amine, *N*-(2-aminoéthyl)-1-(4-hydroxyphényl)imidazo[1,2-*a*]quinoxalin-4-amine, *N*-(2-aminoéthyl)-1-(2,4-dihydroxyphényl)imidazo[1,2-*a*]quinoxalin-4-amine, *N*-(2-aminoéthyl)-1-(2,3-dihydroxyphényl)imidazo[1,2-*a*]quinoxalin-4-amine, *N*-(2-aminoéthyl)-1-(2-méthoxyphényl)imidazo [1,2-*a*]quinoxalin-4-amine, *N*-(2-aminoéthyl)-1-(3-méthoxyphényl)imidazo[1,2-*a*]quinoxalin-4-amine, *N*-(2-aminoéthyl)-1-(4-méthoxyphényl)imidazo[1,2-*a*]quinoxalin-4-amine, *N*-(2-aminoéthyl)-1-(2,4-diméthoxyphényl)imidazo[1,2-*a*]quinoxalin-4-amine, *N*-(2-aminoéthyl)-1-(2,3-diméthoxyphényl)imidazo[1,2-*a*]quinoxalin-4-amine, *N*-(2-aminoéthyl)-1-(3-éthoxyphényl)imidazo[1,2-*a*]quinoxalin-4-amine, *N*-(2-aminoéthyl)-1-(4-éthoxyphényl)imidazo[1,2-*a*]quinoxalin-4-amine, *N*-(2-aminoéthyl)-1-(3-bromophényl)imidazo[1,2-*a*]quinoxalin-4-amine, *N*-(2-aminoéthyl)-1-(4-bromophényl)imidazo[1,2-*a*]quinoxalin-4-amine, *N*-(2-aminoéthyl)-1-(3-(trifluorométhyl)phényl))imidazo[1,2-*a*]quinoxalin-4-amine, *N*-(2-aminoéthyl)-1-(4-(trifluorométhyl)phényl))imidazo[1,2-*a*]quinoxalin-4-amine, *N*-(2-aminoéthyl)-1-(3-chlorophényl)imidazo[1,2-*a*]quinoxalin-4-amine, *N*-(2-aminoéthyl)-1-(4-chlorophényl)imidazo[1,2-*a*]quinoxalin-4-amine, *N*-(2-aminoéthyl)-1-(3-carboxyphényl)imidazo[1,2-*a*]quinoxalin-4-amine, *N*-(2-aminoéthyl)-1-(4-carboxyphényl)imidazo[1,2-*a*]quinoxalin-4-amine, *N*-(2-aminoéthyl)-1-(3-fluorophényl)imidazo[1,2-*a*]quinoxalin-4-amine, *N*-(2-aminoéthyl)-1-(4-fluorophényl)imidazo[1,2-*a*]quinoxalin-4-amine, *N*-(2-aminoéthyl)-1-(3-cyanophényl)imidazo[1,2-*a*]quinoxalin-4-amine, *N-*(2-aminoéthyl)-1-(4-cyanophényl)imidazo[1,2-*a*]quinoxalin-4-amine, *N*-(2-aminoéthyl)-1-(3 - nitrophényl)imidazo[1,2-*a*]quinoxalin-4-amine, *N*-(2-aminoéthyl)-1-(4-nitrophényl)imidazo[1,2-*a*]quinoxalin-4-amine, *N*-(2-aminoéthyl)-1-furanimidazo[1,2-*a*]quinoxalin-4-amine, 1-phénylimidazo[1,2-*a*]quinoxalin-4-amine, 1-(2-hydroxyphényl)imidazo[1,2-*a*]quinoxaline, 1-(3-hydroxyphényl)imidazo[1,2-*a*]quinoxaline, 1-(4-hydroxyphényl)imidazo[1,2-*a*]quinoxaline, 1-(2,4-dihydroxyphényl)imidazo[1,2-*a*]quinoxalin-4-amine, 1-(2,3-dihydroxyphényl)imidazo[1,2-*a*]quinoxalin-4-amine, 1-(2-méthoxyphényl)imidazo[1,2-*a*]quinoxalin-4-amine, 1-(3-méthoxyphényl)imidazo [1,2-*a*]quinoxalin-4-amine, 1-(4-méthoxyphényl)imidazo [1,2-*a*]quinoxalin-4-amine, 1-(2,4-diméthoxyphényl)imidazo[1,2-*a*]quinoxalin-4-amine, 1-(2,3-diméthoxyphényl)imidazo [1,2-*a*]quinoxalin-4-amine, 1-(3-éthoxyphényl)imidazo[1,2-a]quinoxalin-4-amine, 1-(4-éthoxyphényl)imidazo[1,2-*a*]quinoxalin-4-amine, 1-(3-bromophényl)imidazo[1,2-*a*]quinoxalin-4-amine, 1-(4-bromophényl)imidazo[1,2-*a*]quinoxalin-4-amine, 1-(3-(trifluorométhyl)phényl))imidazo[1,2-*a*]quinoxalin-4-amine, 1-(4-(trifluorométhyl)phényl))imidazo[1,2-*a*]quinoxalin-4-amine, 1-(3-chlorophényl)imidazo[1,2-*a*]quinoxalin-4-amine, 1-(4-chlorophényl)imidazo[1,2-*a*]quinoxalin-4-amine, 1-(3-carboxyphényl)imidazo[1,2-*a*]quinoxalin-4-amine, 1-(4-carboxyphényl)imidazo[1,2-*a*]quinoxalin-4-amine, 1-(3-fluorophényl)imidazo[1,2-*a*]quinoxalin-4-amine, 1-(4-fluorophényl)imidazo[1,2-*a*]quinoxalin-4-amine, 1-(3-cyanophényl)imidazo[1,2-*a*]quinoxalin-4-amine, 1-(4-cyanophényl)imidazo[1,2-*a*]quinoxalin-4-amine, 1-(3-nitrophényl)imidazo[1,2-*a*]quinoxalin-4-amine, 1-(4-nitrophényl)imidazo[1,2-*a*]quinoxalin-4-amine, 1-furanimidazo[1,2-*a*]quinoxalin-4-amine, 4-Chloro-1-phénylimidazo[1,2-*a*]quinoxaline, 4-Chloro-1 -(2-hydroxyphényl)imidazo[1,2-*a*]quinoxaline, 4-Chloro-1 -(3-hydroxyphényl)imidazo[1,2-*a*]quinoxaline, 4-Chloro-1 -(4-hydroxyphényl)imidazo[1,2-*a*]quinoxaline, 4-Chloro-1-(2-méthoxyphényl)imidazo[1,2-*a*]quinoxaline, 4-Chloro-1-(3-méthoxyphényl)imidazo[1,2-*a*]quinoxaline, 4-Chloro-1-(4-méthoxyphényl)imidazo[1,2-*a*]quinoxaline, 4-Chloro-1-(2,4-diméthoxyphényl)imidazo[1,2-*a*]quinoxaline, 4-Chloro-1-(2,3-diméthoxyphényl)imidazo[1,2-*a*]quinoxaline, 4-Méthoxy-1-phénylimidazo[1,2-*a*]quinoxaline, 4-Méthoxy-1-(2-hydroxyphényl)imidazo[1,2-*a*]quinoxaline, 4-Méthoxy-1 -(3-hydroxyphényl)imidazo[1,2-*a*]quinoxaline, 4-Méthoxy-1-(4-hydroxyphényl)imidazo[1,2-*a*]quinoxaline, 4-Méthoxy-1-(2-méthoxyphényl)imidazo[1,2-*a*]quinoxaline, 4-Méthoxy-1-(3-méthoxyphényl)imidazo[1,2- *a*]quinoxaline, 4-Méthoxy-1-(4-méthoxyphényl)imidazo [1,2-*a*]quinoxaline, 4-Méthoxy-1-(2,4-diméthoxyphényl)imidazo [1,2-*a*]quinoxaline, 4-Méthoxy-1-(2,3-diméthoxyphényl)imidazo[1,2-*a*]quinoxaline, et leurs sels physiologiquement acceptables.

Un autre objet de la présente description est l'utilisation d'un composé de formule générale (X) : 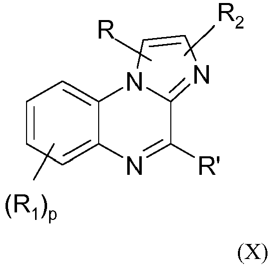 dans laquelle
R, R', R₁ et R₂ représentent indépendamment un atome d'hydrogène, un halogène ou un groupe choisi parmi les radicaux hydroxy, alkyle, alkényle, cycloalkyle, thioalkyle, alcoxy, amino, alkylamino, dialkylamino, acyle, aryle, aralkyle ou un hétérocycle saturé ou non, éventuellement substitués par un ou plusieurs substituants, et les radicaux cyano, nitroso, nitro, -CF₃, - (CH₂)ₙNR₄R₅, -(CH₂)ₙCO-NR₄R₅, -(CH₂)ₙSO₂-NR₄R₅ ou -(CH₂)ₙCO₂R₄, -NH-(CH₂)ₙNR₄R₅, n compris indépendamment entre 0 et 4,
p compris entre 1 et 4 ,
R₄ et R₅ représentent indépendamment un atome d'hydrogène ou groupe choisi parmi les radicaux alkyle en C₁-C₄ linéaire ou ramifié, alkényle linéaire ou ramifié, cycloalkyle en C₃-C₇, acyle, aryle, aralkyle ou un hétérocycle, éventuellement substitués par un ou plusieurs substituants, et ses sels physiologiquement acceptables, pour la préparation d'un médicament destiné au traitement des lymphomes.

De préférence, R, R₁ et R₂ représentent un atome d'hydrogène, un halogène ou un groupe choisi parmi les groupes méthyl, éthyl, propyl, isopropyle, butyle, isobutyle, tertiobutyle, et - (CH₂)_{n"}-(CH=CH)-(CH₂)_{n'''}-CH₃ avec n" et n''' compris indépendamment entre 0 et 4, COOR₄, NR₄R₅ et OR₅, R₄ et R₅ sont définis ci-dessus.
R' représente indépendamment un atome d'hydrogène, un halogène ou un groupe choisi parmi les groupes hydroxy, alcoxy, alkylamino, dialkylamino, un hétérocycle saturé ou non et -NH-(CH₂)ₙNR₄R₅.

Préférentiellement, R₂ est un atome d'hydrogène.

De préférence, R' est un groupe chloro, méthoxy, amino, méthylamino, diméthylamino, éthylamino, diéthylamino, aminométhylamine ou aminoéthylamine. Préférentiellement, R' est - NH-CH₃, -NH₂ ou -NH-(CH₂)₂-NH₂.

Préférentiellement, R est choisi parmi les groupes méthyle, éthyle, propyle, isopropyle, butyle, isobutyle, tertiobutyle et -(CH₂)_{n"}-(CH=CH)-(CH₂)_{n'''}-CH₃ avec n" et n''' compris indépendamment entre 0 et 4.

Dans un mode de réalisation particulier de la description, le composé utilisé répond à la formule générale (XI) : 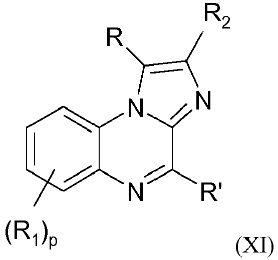 dans laquelle R, R', R₁ et R₂ et p sont définis ci-dessus.

De préférence, la description concerne l'utilisation d'un composé choisi parmi les composés suivants : 1-Isobutylimidazo[1,2-*a*]quinoxalin-4-amine, *N*-méthyl-1-Isobutyl imidazo[1,2-*a*]quinoxalin-4-amine, *N*,*N*-diméthyl-1-Isobutylimidazo[1,2-*a*]quinoxalin-4-amine, *N-*(2-aminoethyl)-1-Isobutylimidazo[1,2-*a*]quinoxalin-4-amine, 4-Chloro-1-Isobutylimidazo[1,2-*a*]quinoxaline et 1-Isobutyl-4-méthoxyimidazo[1,2-*a*]quinoxaline et leurs sels physiologiquement acceptables.

Dans un autre mode de réalisation, l'utilisation concerne un composé de formule générale (XII) : 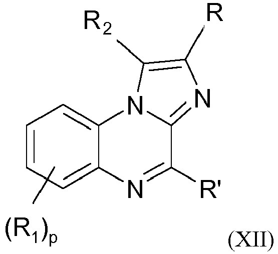 dans laquelle R, R', R₁, R₂ et p sont définis ci-dessus.

Préférentiellement, la description concerne l'utilisation d'un composé choisi parmi les composés suivants : *N*-méthyl-2-isobutylimidazo[1,2-*a*]quinoxalin-4-amine, *N*,*N*-diméthyl-2-isobutylimidazo[1,2-*a*]quinoxalin-4-amine, *N*-(2-aminoethyl)-2-Isobutylimidazo[1,2-*a*]quinoxalin-4-amine 4-Chloro-2-Isobutylimidazo[1,2-*a*]quinoxaline et 2-Isobutyl-4-méthoxyimidazo[1,2-*a*]quinoxaline et leurs sels physiologiquement acceptables.

Préférentiellement, l'invention se rapporte aux composés selon l'invention pour utilisation comme médicament.

Plus préférentiellement, l'invention se rapporte aux composés selon l'invention pour utilisation comme médicament pour le traitement des cancers.

Encore plus préférentiellement, l'invention se rapporte aux composés selon l'invention pour le traitement des mélanomes ou des lymphomes.

L'invention a aussi pour objet une composition pharmaceutique comprenant un composé tel que défini ci-dessus et un véhicule pharmaceutique appropriée.

Un autre objet de la présente invention est un produit comprenant un composé tel que défini ci-dessus et un autre agent actif comme produit de combinaison pour une utilisation simultanée, séparée ou étalée dans le temps en thérapie.

L'invention se rapporte aussi à l'utilisation d'un composé tel que défini ci-dessus pour la préparation d'un médicament destiné au traitement des cancers. Plus préférentiellement, à l'utilisation d'un composé selon l'invention pour la préparation d'un médicament destiné au traitement des mélanomes ou des lymphomes.

Dans un mode de réalisation avantageux, les composés selon l'invention sont utilisés en association avec au moins un autre agent actif.

L'invention concerne aussi des méthodes de traitement thérapeutique des cancers comprenant l'administration d'une quantité efficace d'un composé (ou d'un sel physiologiquement acceptable d'un composé) selon l'invention à un individu. De préférence, l'invention se rapporte à des méthodes de traitement thérapeutique des mélanomes et des lymphomes.Par halogène, on entend en particulier selon la présente invention les halogènes suivants: F, Cl, Br et I.

Par alkyle, on entend en particulier les radicaux alkyles linéraires ou ramifiés, en particulier les radicaux alkyles en C1, C2, C3, C4, C5 ou C6, en particulier les radicaux méthyle, éthyle, n-propyle, *i*-propyle, *n*-butyle, *i*-butyle ou *t*-butyle. Cette définition s'applique également aux parties alkyles des radicaux cycloalkyl, alcoxy, acyle, aralkyle, alkylamino, thioalkyle.

Par alkényle, on entend de préférence une chaîne hydrocarbonée, monovalente, insaturée et comprenant au moins une double liaison, linéaire ou ramifiée, comportant de 2 à 6 atomes de carbone, dont des éléments représentatifs sont par exemple les groupes vinyle, 1-propényle, 2-propényle, isoprenyle, butényle, pentenyle, hexenyle.

Par cycloalkyle, on entend avantageusement les cycloalkyles en C3-C7, plus particulièrement les radicaux cyclopropyle, cyclobutyle, cyclopentyle, cyclohexyle et cycloheptyle.

Par aryle, on entend de préférence un ou plusieurs cycles aromatiques ayant 6 à 10 atomes de carbone, pouvant être accolés ou fusionnés, en particulier le phényle. Cette définition s'applique également à la partie aryle des radicaux aralkyles. Le groupe aralkyle est de préférence (CH₂)ₙ-phényle dans lequel n est compris entre 0 et 4.

Par amino ou amine, on entend une amine primaire, secondaire ou tertiaire.

Par hétérocycle, on entend avantageusement un cycle en C3-C7 contenant au moins un hétéroatome choisi parmi l'azote, l'oxygène ou le soufre, en particulier les hétérocycles sont choisis parmi thiényle, furyle, quinolinyle, indolyle, pyrazole, pyrrole, pyridine, pyrimidine, imidazole.

Par « cancer », on entend toutes les formations néoplasiques malignes, quelle qu'en soit la nature histologique. Il existe deux grandes catégories de tumeurs malignes : les carcinomes, d'origine épithéliale, et les sarcomes, d'origine conjonctive. Les tumeurs malignes sont formées de cellules atypiques, envahissantes ou disséminantes, caractérisées généralement par un pouvoir d'accroissement autonome, une délimitation imprécise, une capacité d'envahissement des tissus et vaisseaux voisins et une tendance à disséminer par la production de métastases. On citera notamment les cancers du sein, de la prostate, des poumons, de l'oesophage, de la peau, de la vessie, de l'estomac, du foie, de l'utérus, du côlon et du rectum.

Par « mélanome », on entend une tumeur maligne qui se développe aux dépens des tissus pigmentés, ceux de la peau ou de l'oeil plus spécialement.

Par « lymphome », on entend toute tumeur, généralement maligne, due à une prolifération des cellules du tissu lymphoïde, se développant surtout au niveau de la rate ou des ganglions.

Par sel pharmaceutiquement acceptable on entend de préférence selon l'invention un sel d'acide pharmaceutiquement acceptable, c'est-à-dire avec tout acide non toxique, y compris les acides organiques et inorganiques. De tels acides incluent l'acide acétique, benzènesulfonique, benzoïque, citrique, éthanesulfonique, fumarique, gluconique, glutamique, bromhydrique, chlorhydrique, lactique, maléique, malique, mandélique, méthanesulfonique, mucique, nitrique, pamoïque, pantothénique, phosphorique, succinique, sulfurique, tartrique et paratoluènesulfonique.

L'invention concerne une composition pharmaceutique comprenant un composé tel que défini ci-dessus et un véhicule pharmaceutique approprié.

Ces compositions peuvent être formulées pour l'administration aux mammifères, y compris l'homme. La posologie varie selon le traitement et selon l'affection en cause. Ces compositions sont réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants.

Les composés selon l'invention peuvent être employés en thérapie seuls, ou en combinaison avec au moins un autre agent actif. Ces autres agents actifs sont en particulier choisis parmi les actifs appropriés pour le traitement des cancers. Il peut s'agir d'adjuvants permettant d'améliorer l'activité des composés selon l'invention, ou encore d'autres actifs connus pour leur emploi dans le traitement des dites affections. De tels agents actifs sont bien connus de l'homme du métier, disponibles dans le commerce ou encore décrits dans des ouvrages de référence comme Le Dictionnaire Vidal, édité avec mises à jours chaque année, en particulier les agents actifs regroupés sous les familles pharmacothérapeutiques « Cancérologie Hématologie ».

La présente invention concerne donc également un produit comprenant un composé selon l'invention et un autre agent actif comme produit de combinaison pour une utilisation simultanée, séparée ou étalée dans le temps en thérapie, et en particulier dans le traitement des cancers.

Les composés selon l'invention peuvent être préparés selon les différents modes de préparation exposés ci-dessous et dans les exemples.
Synthèse 1 (référence) : Les dérivés imidazo[1,2-*a*]quinoxalines ont été obtenus par condensation entre un α-aminoalcool et une quinoxaline, suivi d'une cyclisation intramoléculaire et de différentes substitutions nucléophiles. Les α-cyanoalcools (le 2-hydroxy-4-méthylpentanenitrile ou le 2-hydroxy-4-phénylbutanenitrile) résultent de la réaction entre l'isovaleraldéhyde en série **a** ou le 3-phénylpropionaldéhyde en série **b**, et le cyanure de sodium. Ils sont ensuite réduits à l'aide de l'hydrure d'aluminium lithium pour donner les α-aminoalcools correspondants (soit le 1-amino-4-méthylpentan-2-ol **1a**, soit le 1-amino-4-phénylbutan-2-ol **1b**). Les **1a** et **1b** sont ensuite condensés avec la 2,3-dichloroquinoxaline en présence de triéthylamine dans le dioxane pour former le 1-[(3-chloroquinoxalin-2-yl)amino]-4-méthylpentan-2-ol **2a** et le 1-[(3-chloroquinoxalin-2-yl)amino]-4-phénylbutan-2-ol **2b**. Une oxydation de ces deux composés par le complexe triméthylamine trioxide de soufre est nécessaire pour permettre leur cyclisation intramoléculaire et obtenir respectivement les dérivés **4a** et **4b.** Enfin, une substitution nucléophile est effectuée sur les des deux dérivés chlorés pour donner **5b, 6a, 6b, 7a, 7b, 8b** selon la nature du groupement R (voir schéma ci-dessous).
Synthèse 2 (invention) : Le dimère **9** diimidazo[1,2-*a*]:[1',5'-*d*]pipérazine-5,10-dione résulte de la condensation bimoléculaire de l'acide imidazo-2-carboxylique en présence de chlorure de thionyle. Il est ensuite couplé à l'orthofluoroaniline pour donner l'intermédiaire **10.** La construction du composé tricyclique **11,** se fait par cyclisation intramoléculaire de **10,** en présence d'hydrure de sodium dans le diméthylacétamide. Une étape de chloration suivit d'une substitution avec une méthylamine conduisent au composé **13.** Le composé **13** est ensuite bromé par le N-bromosuccinimide pour donner le composé bromé **14,** qui sera à son tour substitué par des groupements alkyl via les réactions de suzuki pour donner les composés **15a à 15n** (voir schéma ci-dessous).

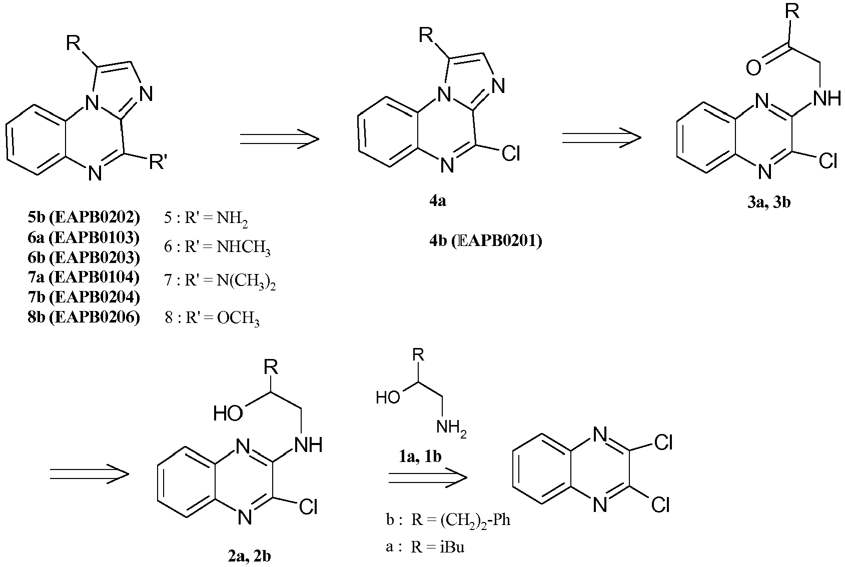

### Synthèse 1 de la série imidazo[1,2-a]quinoxaline (référence)

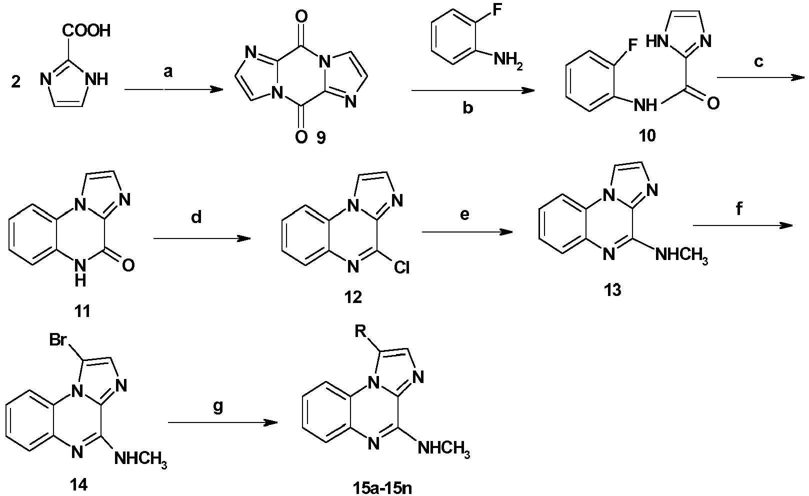

### Synthèse 2 de la série 1H-imidazo[1,2-a]quinoxaline (invention)

Réactifs et conditions: (a) SOCl₂ reflux, 18 h; (b) NaHMDS, THF, 5 h; (c) NaH, DMA, reflux, 10 h; (d) POCl₃, reflux, 6 h; (e) EtOH, NHCH₃ ,20 h, rt; (f) NBS, CHCl₃, reflux, 2h; (g) arylboronic acid (R₁-B(OH)₂), Pd(PPh₃)₄, Na₂CO₃, DME, MW (140°C, 20 min).

### FIGURES

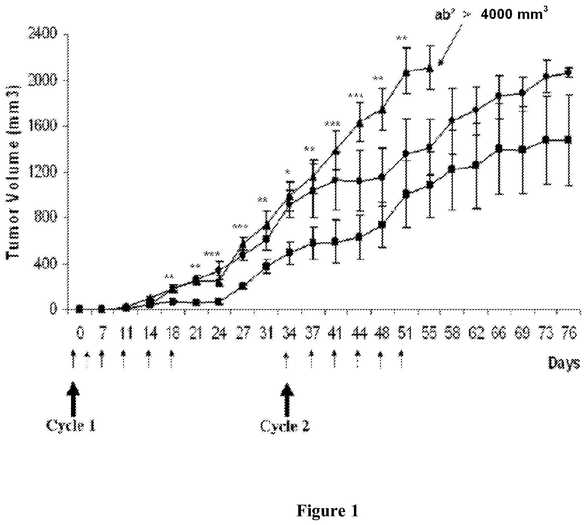

**Figure 1** **(référence) :** Les effects de la fotémustine (20 mg/kg une fois par semaine pendant 3 semaines) et de **EAPB0203** (20 mg/kg deux fois par semaine pendant 3 semaines) sur la croissance tumorale chez des souris SWISS athymiques xénogreffées par la lignée cellulaire M4Be du mélanome humain.
   , administrations de fotémustine et de **EAPB0203;** , administrations de **EAPB0203.** ▲, contrôle; **■, EAPB0203; ●,** fotémustine. Les données sont les résultats d'une seule expérience réalisée sur six souris par groupe. Les résultats sont représentés en moyenne± S.E.M. Différence significative entre les trois groupes de traitement: *, P<0.05 ; **, 0.002<P<0.009; ***, P ≤ 0.001.

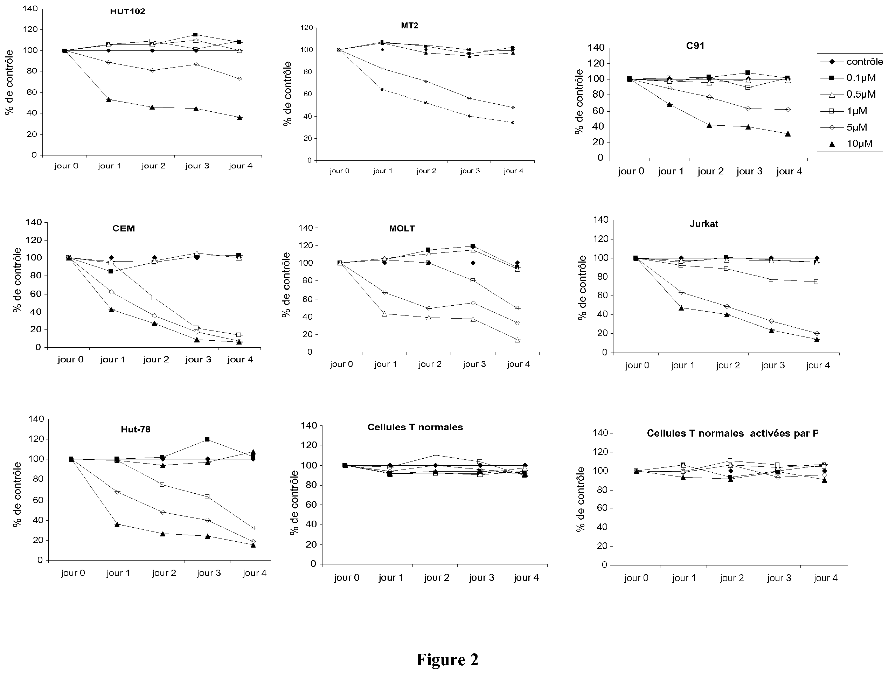

**Figure 2** **(référence)** : Les lignées cellulaires humaines malignes HTLV-I-positives et HTLV-I-négatives sont sensibles à **l'EAPB0203**, mais les lymphocytes T normaux activés ou non sont résistants. Effets de **l'EAPB0203** sur la croissance des lignées T humaines non infectées par le virus HTLV-I (CEM, Jurkatt, Molt-4 et HuT-78), des lignées T humaines HTLV-I-positives (HuT-102 et C8166, et MT2), et des lymphocytes normaux au repos ou activés par PHA. Les PBMC normaux activés ont été complétés avec 2% PHA. **EAPB0203** a été ajouté aux concentrations indiquées en mole/L pour 24-96h. La croissance cellulaire est analysée avec le kit non radioactif de prolifération cellulaire 'CellTiter 96®'. Les résultats sont exprimés par rapport au pourcentage du contrôle (0.1% DMSO) et représentent la moyenne des résultats obtenus en trois expériences indépendantes.

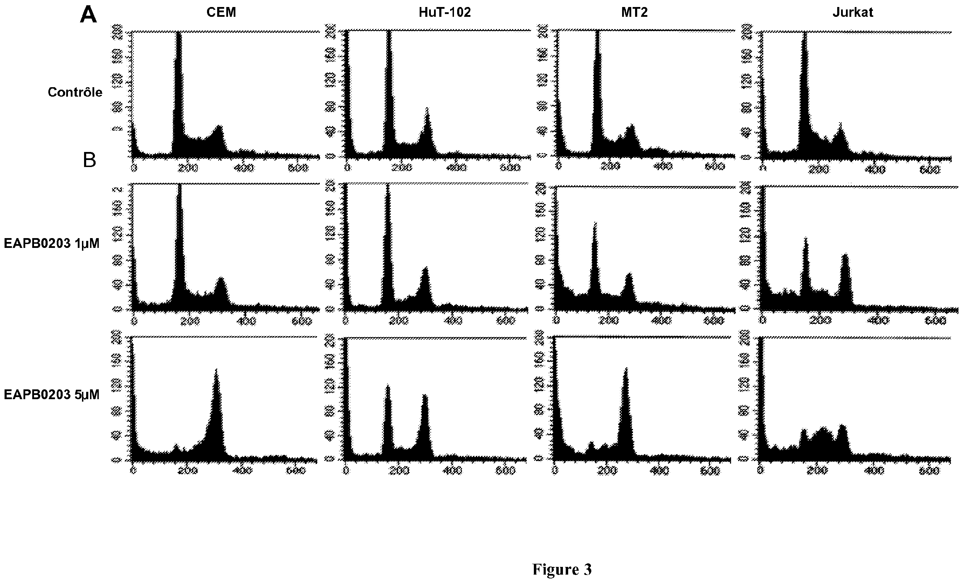

**Figure 3** **(référence) : EAPB0203** induit l'arrêt du cycle cellulaire en phase G2/M dans les lignées humaines de cellules T HTLV-I-positives et HTLV-I-négatives. (a) Les effets de l'**EAPB0203** sur la distribution du cycle cellulaire des cellules CEM, de HuT-102, de MT2, et de Jurkatt. Les cellules traitées par **EAPB0203** ont été marquées avec l'iodure de propidium (50 mg/ml) et l'analyse de cycle cellulaire a été exécutée par cytométrie de flux FACScan. (b) Le pourcentage pre-G1 représente les cellules apoptotiques. (c) Les cellules en cycle, la somme de phases (S+G2/M), sont représentées en pourcentage des cellules non-apoptotiques. Les résultats sont représentants de deux expériences indépendantes.

### EXEMPLES

### 1) Synthèse des composés imidazo[1,2-a]quinoxalines

### 2-Hydroxy-4-méthylpentanenitrile (référence)

10 g (116 mmol) d'isovaléraldéhyde sont ajoutés pendant 2 ou 3 minutes à une solution de NaHSO₃ à 37% aqueux (25 ml, 116 mmol), à 0 °C, et sous agitation. Un précipité blanc de bisulfite se forme presque immédiatement. Une solution de NaCN (5.7 g, 116 mmol) dans H₂O (30 ml) est alors ajoutée goutte-à-goutte, pendant 45 min. L'agitation est maintenue pendant 18 h à température ambiante; pendant ce temps le précipité a été solubilisé et deux couches non-miscibles se sont formées. Le mélange est extrait à Et₂O (30 ml). Les phases éthérées sont rassemblées, séchées par Na₂SO₄ et évaporées pour donner une huile jaune utilisée sans autre purification (12.21 g, 93%) ; ¹H RMN (100 MHz, CDCl₃) δ: 0.88 (d, J=6 Hz, 6H), 1.51-1.98 (m, 3H, H-3+H-4), 4.20 (s, Br, OH), 4.45 (t, J=7 Hz, 1H) ; ¹³C RMN (25 MHz, DMSO-*d₆*) δ: 21.6, 22.1, 23.8, 43.1, 58.3, 121.3. Analyse calculée pour C₆H₁₁NO : C, 63.68 ; H, 9.80 ; N, 12.38. Expérimental : C, 63.56 ; H, 9.75 ; N, 12.42.

### 2-Hydroxy-4-phénylbutanenitrile (référence)

Le 2-Hydroxy-4-phénylbutanenitrile est préparé à partir du 3-phénylpropionaldehyde d'après le protocole décrit pour le 2-Hydroxy-4-méthylpentanenitrile; (11.14 g, 83.2 mmol) 3-phénylpropionaldehyde, NaHSO₃ (17.7 ml de solution à 37%, 83 mmol), (4.08 g, 83.2 mmol) de NaCN dans H₂O (18 ml). Le produit, une huile jaune, est utilisée sans autre purification (12.4 g, 93%) ; ¹H RMN (100 MHz, CDCl₃) δ : 2.02-2.25 (m, 2H), 2.76-2.91 (m, 2H), 3.98 (s, 1H), 4.39 (t, J=8 Hz, 1H), 7.26 (d, J=3 Hz, 5H) ; ¹³C RMN(25 MHz, CDCl₃) δ : 30.49, 36.37, 59.92, 120.04, 126.28, 128.28, 128.49, 139.60. Analyse calculée pour C₁₀H₁₁NO : C, 74.51 ; H, 6.88 ; N, 8.69. Expérimental : C, 74.38 ; H, 6.97 ; N, 8.42.

### 1-Amino-4-méthylpentan-2-ol(1a) (référence)

Une solution de 2-Hydroxy-4-méthylpentanenitrile (12.13 g, 107 mmol) dissoute dans Et₂O (50 ml), est ajoutée goutte-à-goutte, pendant 45 minutes, à léger reflux, dans une solution de LiAlH₄ (8.12 g, 214 mmol) dans Et₂O (160 ml)sous agitation. Après l'ajout, le mélange est encore chauffé au reflux pendant 90 mn. Après refroidissement à 0-5 °C, l'excès de LiAlH₄ est neutralisé par addition goutte-à-goutte de H₂O (8 ml), de NaOH aqueux à 15% (8 ml) et de H₂O (40 ml). Le mélange est agité jusqu'à ce que tout le LiAlH₄ ait été neutralisé et qu'un précipité blanc se soit formé. Le mélange est filtré et le précipité est lavé avec Et₂O. La phase organique est séchée (par des granules de KOH) et évaporée à sec sous pression réduite pour obtenir une huile orange qui est utilisée sans autre purification (11.78 g, 94%) ; ¹H RMN (100 MHz, CDCl₃) δ : 0.74 (d, J=7 Hz, 6H), 1.04 (m, 2H, H-3), 1.56 (m, J=7 Hz, 1H, H-4), 2.41 (m, 5H, H-1, l'OH, NH₂), 3.40 (m, J=4 Hz, 1H, H-2) ; ¹³C RMN (25 MHz, DMSO-*d₆*) δ : 21.90, 23.20, 24.30, 43.80, 47.80, 69.90. Analyse calculée pour C₆H₁₅NO : C, 61.49 ; H, 12.90 ; N, 11.95. Expérimental : C, 61.70 ; H, 12.53 ; N, 11.67.

### 1-Amino-4-phénylbutan-2-ol (1b) (référence)

**1b** est préparé à partir du 2-Hydroxy-4-phénylbutanenitrile d'après le protocole décrit pour **la** ; LiAlH₄ (6.2 g, 163 mmol), Et₂O (120 ml), 2-Hydroxy-4-phénylbutanenitrile (12.4 g, 77 mmol), H₂O (6.2 ml), NaOH aqueux à 15% (6.2 ml) et H₂O (18 ml). Le produit, une huile orange, est utilisée sans autre purification (11 g, 87%) ; ¹H RMN (100 MHz, CDCl₃) δ : 1.80 (m, 3H), 2.65 (m, 4H), 3.62 (m, 3H), 7.21 (s, 5H) ; ¹³C RMN (25 MHz, CDCl₃) δ : 33.98, 36.25, 70.92, 125.46, 128.04, 141.74. Analyse calculée pour C₁₀H₁₅NO : C, 72.69 ; H, 9.15 ; N, 8.48. Expérimental : C, 72.45 ; H, 9.33 ; N, 8.24.

### 1-[(3-Chloroquinoxalin-2-yl)amino]-4-méthylpentan-2-ol (2a) (référence)

La 2,3-dichloroquinoxaline (18.05 g, 90.7 mmol) est ajoutée à une solution de **la** brut (11.7 g, 99.8 mmol) et Et₃N (19 ml, 13.8 g, 136 mmol) dans du dioxane (210 ml). La solution obtenue est chauffée à reflux (sous N₂) pendant 6 h, elle est ensuite refroidie à température ambiante. Et₃N, HCl est éliminée par filtration et le filtrat est concentré sous pression réduite. Le résidu orange-foncé est purifié par chromatographie sur colonne de silice, éluant : CH₂Cl₂/MeOH (100:0→99:1), pour obtenir un solide jaune (12.14 g, 48%) ; ¹H RMN (100 MHz, CDCl₃) δ : 0.72 (d, 3H), 0.78 (d, 3H), 1.60 (m, 3H), 3.68 (m, 4H), 5.82 (t, 1H), 7.58 (m, 4H) ; ¹³C RMN (25 MHz, CDCl₃) δ : 22.17, 23.35, 24.60, 44.32, 48.62, 69.67, 125.30, 125.62, 127.91, 130.36, 136.52, 137.92, 140.59, 148.62. Analyse calculée pour C₁₄H₁₈N₃OCl : C, 60.10 ; H, 6.49 ; N, 15.02. Expérimental : C, 59.87 ; H, 6.62 ; N, 15.25.

### 1-[(3-Chloroquinoxalin-2-yl)amino]-4-phénylbutan-2-ol (2b) (référence)

**2b** est obtenu à partir de **1b** d'après le protocole décrit pour 2a ; 2,3-dichloroquinoxaline (9.95 g, 50 mmol), **1b** (9.02 g, 55 mmol) dans du dioxane (250 ml) avec Et₃N (7.57 g, 75 mmol). Le produit brut est purifié par chromatographie sur colonne de silice, éluant : CH₂Cl₂/MeOH (98:2) pour obtenir un solide jaune (10 g, 61%) ; ¹H RMN (100 MHz, CDCl₃) δ : 1.76-1.97 (m, 2H), 2.70-2.90 (m, 2H), 3.53-4.05 (m, 4H), 5.96 (t, J=5 Hz, 1H), 7.23-7.82 (m, 9H). Analyse calculée pour C₁₈H₁₈N₃OCl: C, 65.95 ; H, 5.53 ; N, 12.82. Expérimental : C, 66.16 ; H, 5.43 ; N, 12.58.

### 1-[(3-Chloroquinoxalin-2-yl)amino]-4-méthylpentan-2-one (3a) (référence)

Un mélange de **2a** (6.96 g, 24.8 mmol), de 28.4 ml Et₃N et de complexe de triméthylamine de trioxyde soufré (7.9 g, 56.8 mmol) dans 28.4 ml de DMSO, est agité toute une nuit (sous N₂) à température ambiante, puis addition d'eau glacée (50 ml). La phase aqueuse est extraite au CH₂Cl₂ (3×30 ml). Les phases organiques rassemblées sont séchées (CaCl₂) et évaporées sous pression réduite. Les résidus sont purifiés par chromatographie sur colonne de silice, éluant : C₆H₁₂/Et₂O (85:15) pour donner un solide beige (4.18 g, 61%) ; ¹H RMN (100 MHz, CDCl₃) δ : 0.93 (s, 3H), 1.00 (s, 3H), 2.34 (m, 1H), 2.40 (s, 2H), 4.38 (d, 2H), 6.39 (t, 1H), 7.59 (m, 4H) ; ¹³C RMN (25 MHz, CDCl₃) δ : 22.56, 25.01, 49.28, 51.55, 125.34, 125.87, 127.98, 130.14, 136.65, 137.92, 140.99, 147.30, 205.01. Analyse calculée pour C₁₄H₁₆N₃OCl : C, 60.54 ; H, 5.81 ; N, 15.13. Expérimental : C, 60.32 ; H, 6.06 ; N, 14.95.

### 1-[(3-Chloroquinoxalin-2-yl)amino]-4-phénylbutan-2-one (3b) (référence)

**3b** est préparé à partir de **2b** d'après le protocole décrit pour **3a** ; **2b** (4.7 g, 14.4 mmol), 14.4 ml DMSO, 14.4 ml Et₃N, et Me₃N.SO₃ (4 g, 28.8 mmol). Le produit est purifié par chromatographie sur colonne de silice, éluant : C₆H₁₂/Et₂O (80:20) pour donner un solide jaune (4.51 g, 96%) ; ¹H RMN (100 MHz, CDCl₃) δ : 2.82-3.01 (m, 4H), 4.38 (d, J=5 Hz, 2H), 6.4 (m, 1H), 7.23-7.84 (m, 9H). Analyse calculée pour C₁₈H₁₆N₃OCl : C, 66.36 ; H, 4.95 ; N, 12.90. Expérimental : C, 66.53 ; H, 5.23 ; N, 12.78.

### 4-Chloro-1-Isobutylimidazo[1,2-a]quinoxaline (4a) (EAPB0101) (référence)

**4a** (16.39 g, 23 mmol) est solubilisé dans un mélange d'anhydride trifluoroacétique (100 ml) et d'acide trifluoroacétique (1 ml), et agité sous azote pendant 24 h à température ambiante. Le solvant est ensuite évaporé sous pression réduite et le résidu est solubilisé dans du dichlorométhane (300 ml). La phase organique est lavée avec une solution à 5% de NaHCO₃ (75 ml) puis avec de l'eau, séchée par Na₂SO₄ et concentrée sous vide pour donner une huile orange. Le produit est purifié par chromatographie sur colonne de silice, éluant C₆H₁₂/Et₂O (95:5) pour donner un solide beige (5.04 g, 84%); 1H NMR (100 MHz, CDCl₃) d: 1.07 (d, J=6-7 Hz, 6H), 2.16 (m, 1H), 3.11(d, J=6-7 Hz, 2H), 7.53-7.64 (m, 3H), 7.96-8.16 (m, 2H); 13C NMR (25 MHz, CDCl3) d: 22.41, 26.88, 36.66, 115.39, 126.50, 128.41, 128.94, 130.04, 132.02, 134.37, 135.61, 136.60, 143.66. Anal. calcd for C14H14N3Cl: C, 64.74; H, 5.43; N, 16.18. Found: C, 64.66; H, 5.55; N, 15.86. **4-Chloro-1-(2-phényléthyl)imidazo[1,2-*a*]quinoxaline (4b) (EAPB0201) (référence)**

**4b** est préparé à partir de **3b** d'après le protocole décrit pour **4a** ; **3b** (4 g, 12.2 mmol), anhydride trifluoroacetique (100 ml), acide trifluoroacetique (4 ml). Le produit est purifié par chromatographie sur colonne de silice, éluant : CH₂Cl₂/MeOH (98:2) pour donner un solide jaune (2.5 g, 66%) ; ¹H RMN (100 MHz, CDCl₃) δ : 3.20-3.31 (m, 2H), 3.53-3.68 (m, 2H), 7.32 (s, 5H), 7.54-7.63 (m, 3H), 7.99-8.31 (m, 2H) ; ¹³C RMN (25 MHz, CDCl₃) δ : 30.05, 33.80, 115.34, 126.54, 126.66, 128.19, 128.46, 128.70, 129.94, 129.20, 133.00, 139.72. Analyse calculée pour C₁₈H₁₄N₃Cl : C, 70.24 ; H, 4.58 ; N, 13.65. Expérimental : C, 70.04 ; H, 4.96 ; N, 13.82.

### 1-Isobutylimidazo[1,2-a]quinoxalin-4-amine (5a) (EAPB0102) (référence)

**4a** (1 g, 3.85 mmol) est chauffé à 120°C pendant 4 h en présence d'une solution aqueuse d'ammoniaque (60 ml à 30% (poids/volume), 0.5 mmol). Le mélange de la réaction est refroidi à température ambiante puis filtré. Le précipité est lavé avec H₂O (10 ml), dissous dans CH₂Cl₂ (25 ml), et la phase organique est séchée (Na₂SO₄) puis concentrée à pression réduite. Le produit brut est purifié par chromatographie sur colonne de silice, éluant : CH₂Cl₂/MeOH (90:10) pour donner un solide jaune (0.75 g, 81%); ¹H RMN (100 MHz, DMSO-*d₆*) δ : 2.07 (m, J=6-7 Hz, 1H), 3.09 (d, J=6-7 Hz, 2H), 7.08-8.05 (m, 7H). Analyse calculée pour C₁₄H₁₆N₄ : C, 69.97 ; H, 6.71 ; N, 23.32. Expérimental : C, 70.13 ; H, 6.97 ; N, 23.07.

### 1-(2-phényléthyl)imidazo[1,2-a]quinoxalin-4-amine (5b) (EAPB0202) (référence)

**5b** est préparé à partir de **4b** d'après le protocole décrit pour **5a** ; **4b** (0.8 g, 2.6 mmol) ; solution aqueuse d'ammoniaque (48 ml à 30% (poids/volume), 0.4 mmol). Un solide jaune est obtenu après purification par chromatographie sur colonne comme indiqué pour **5a** (0.465 g, 62%) ; ¹H RMN (100 MHz, CDCl₃) δ : 3.10-3.24 (m, 2H), 3.48-3.63 (m, 2H), 5.74 (s, 2H), 7.20-7.48 (m, 8H), 7.69 (d, J=7.2 Hz, 1H), 8.05 (d, J=7.9 Hz, 1H) ; ¹³C RMN (25 MHz, CDCl₃) δ : 29.75, 34.00, 115.01, 123.09, 125.94, 126.33, 126.78, 128.08, 128.46, 130.51, 130.89, 137.50, 140.04, 148.44. Analyse calculée pour C₁₈H₁₆N₄ : C, 74.98 ; H, 5.59 ; N, 19.43. Expérimental : C, 74.86 ; H, 5.75 ; N, 19.37.

### 1-Isobutyl-N-méthylimidazo[1,2-a]quinoxalin-4-amine (6a) (EAPB0103) (référence)

Une solution aqueuse de méthylamine (0.6 ml à 40% (poids/volume), 6.93 mmol) est ajoutée goutte-à-goutte à une solution de **4a** (0.6 g, 2.31 mmol) dans EtOH absolu (15 ml) à température ambiante et sous agitation. Après 40 h, une autre portion de solution aqueuse de méthylamine (0.6 ml à 40% (poids/volume), 6.93 mmol) est ajoutée et maintenue sous agitation pendant 3 heures supplémentaires. Le solvant est évaporé sous pression réduite et le résidu obtenu est dissous dans CH₂Cl₂ (50 ml). La phase organique est successivement lavée avec NaHCO₃ à 5% (30 ml) et H₂O (30 ml), séchée (Na₂SO₄) et concentrée sous pression réduite. Le produit est purifié sur chromatographie sur colonne de silice, éluant : C₆H₁₂/EtO Ac (70:30) pour obtenir un solide couleur crème (0.49 g, 83%) ; ¹H RMN (100 MHz, CDCl₃) δ : 1.04 (d, J=6.2 Hz, 6H), 1.90-2.35 (m, 1H), 3.03 (d, J=7.0 Hz, 2H), 3.20 (d, J=4.9 Hz, 3H), 6.10-6.40 (m, 1H), 7.10-7.55 (m, 3H), 7.60-8.00 (m, 2H) ; ¹³C RMN (25 MHz, CDCl₃) δ : 22.43, 26.93, 27.37, 36.65, 115.15, 122.49, 125.93, 126.63, 127.38, 130.43, 131.32, 134.01, 138.23, 148.41. Analyse calculée pour C₁₅H₁₈N₄ : C, 70.84 ; H, 7.13 ; N, 22.03. Expérimental : C, 71.12 ; H, 7.44 ; N, 22.21.

### N-Méthyl-1-(2-phényléthyl)imidazo[1,2-a]quinoxalin-4-amine (6b) (EAPB0203) (référence)

**6b** est préparé à partir du **4b** d'après le protocole décrit pour **6a** ; solution aqueuse de méthylamine (0.260 ml à 40% (poids/volume), 3 mmol), **4b** (0.307 g, 1 mmol). Le produit brut est purifié par chromatographie sur colonne, éluant : CH₂Cl₂/MeOH (90:10) pour obtenir un solide jaune (0.2 g, 66%) ; ¹H RMN (100 MHz, CDCl₃) δ : 3.07-3.22 (m, 5H), 3.42-3.58 (m, 2H), 6.56 (d, J=5.6 Hz, 1H), 7.12-7.45 (m, 7H), 7.73 (d, J=9.1 Hz, 1H), 7.98 (d, J=7.9 Hz, 1H) ; ¹³C RMN (25 MHz, CDCl₃) δ : 27.27, 29.76, 33.96, 114.93, 122.28, 125.79, 126.33, 127.05, 129.16, 129.49, 129.79, 130.51, 133.72, 133.97, 140.24, 149.14. Analyse calculée pour C₁₉Hl₈N₄ : C, 75.47 ; H, 6.00 ; N, 18.43. Expérimental : C, 75.68 ; H, 6.15 ; N, 18.51.

### 1-Isobutyl-N,N-diméthylimidazo[1,2-a]quinoxalin-4-amine (7a) (EAPB0104) (référence)

**7a** est préparé à partir de **4a** d'après le protocole décrit pour **6a** ; solution aqueuse de diméthylamine (1.3 ml à 40% (poids/volume), 11.55 mmol), **4a** (1.03 g, 3.85 mmol) dans EtOH absolu (10 ml). Le produit brut est purifié par chromatographie sur colonne de silice, éluant : CH₂Cl₂/MeOH (97:3) pour donner un solide blanc (0.8 g, 78%) ; ¹H RMN (100 MHz, CDCl₃) δ : 1.03 (d, J=6-7 Hz, 6H), 2.16 (m, J=6-7 Hz, 1H), 3.03 (d, J=6-7 Hz, 2H), 3.58 (s, 6H), 7.15-7.41 (m, 3H), 7.62-7.72 (m, 1H), 7.86-7.95 (m, 9H) ; ¹³C RMN (25 MHz, CDCl₃) δ : 22.42, 26.75, 36.89, 40.00, 114.86, 122.05, 125.80, 126.50, 126.80, 126.98, 129.51, 131.08, 134.74, 137.74, 149.15. Analyse calculée pour C₁₆H₂₀N₄ : C, 71.61 ; H, 7.51 ; N, 20.88. Expérimental : C, 71.77 ; H, 7.23 ; N, 20.64.

### N,N-Diméthyl-1-(2-phényléthyl)imidazo[1,2-a]quinoxalin-4-amine (7b) (EAPB0204) (référence)

**7b** est préparé à partir de **4b** d'après le protocole décrit pour **7a** ; **4b** (0.32 g, 1 mmol), solution aqueuse de diméthylamine (0.44 ml à 40% (poids/volume), 3.90 mmol). Le produit est purifié par chromatographie sur colonne comme indiqué pour **7a** et on obtient un solide beige (0.2 g, 65%) ; ¹H RMN (100 MHz, CDCl₃) δ : 3.09-3.26 (m, 2H), 3.42-3.54 (m, 2H), 3.59 (s, 6H), 7.14-7.42 (m, 8H), 7.70 (d, J=6.5 Hz, 1H), 8.00 (d, J=6.9 Hz, 1H) ; ¹³C RMN (25 MHz, CDCl₃) δ : 30.41, 34.30, 40.12, 114.99, 125.25, 126.02, 126.58, 127.07, 128.43, 128.75, 129.89, 130.02, 134.84, 137.82, 140.57, 149.21. Analyse calculée pour C₂₀H₂₀N₄ : C, 75.92 ; H, 6.37 ; N, 17.71. Expérimental : C, 75.82; H, 6.15 ; N, 17.57.

### 4-Méthoxy-1-(2-phényléthyl)imidazo[1,2-a]quinoxaline (8b) (EAPB0206) (référence)

Une solution méthanolique de méthylate de sodium a été préparée à partir du sodium (0.23 g, 10 mmol) et du méthanol sec (70 ml). **4b** (1 g, mmol 4.9) a été additionné et la solution résultante a été chauffée au reflux pendant 2h et laissée sous agitation pendant 24h à température ambiante. Le mélange réactionnel a été évaporé à sec sous vide et le résidu a été dissous dans du dichlorométhane (150 ml), lavé avec du chlorure de sodium (100 ml), de l'eau (100 ml), et évaporé pour donner le produit brut. Ce dernier a été purifié par chromatographie sur colonne, éluant : dichlorométhane/méthanol (98/2), pour obtenir le composé **8b** (1.3 g, 95%) ; ¹H-NMR (200 MHz, CDCl₃) δ : 8.77 (d, 1H, H1), 8.27 (dd, 1H, H9), 7.81 (dd, 1H, H6), 7.74 (d, 1H, H2), 7.56 (m, 2H, H7+H8), 4.15 (s, 3H, OCH3). Analyse calculée pour C₁₉H₁₇N₃O : C, 75.23 ; H, 5.65 ; N, 13.85. Expérimental : C, 75.19 ; H, 5.69 ; N, 13.88.

### Diimidazo[1,2-a;1',2'-d]pyrazine-5,10-dione (9) (invention)

L'acide 2-imidazocarboxylique (2.5 g, 22.3 mmol) est mis en suspension dans du chlorure de thionyle (40 ml). Le mélange est porté à reflux, sous agitation pendant 18h. Obtention d'un mélange laiteux qui vire au marron orangé. Le milieu réactionnel est refroidi, puis filtré sur fritté. Le solide jaune ainsi obtenu est lavé au toluène, et séché sous vide (3.52 g, 84.4%) ; ¹H RMN (300MHz, DMSOd₆) δ : 8.1 (s, 2H, ArH) ; 7.45 (s, 2H, ArH). Analyse calculée pour C₈H₄N₄O₂ : C, 51.07 ; H, 2.14 ; N, 29.78. Expérimental : C, 51.15 ; H, 2.21 ; N, 29.46.

### N-(2-Fluorophényl)-1H-imidazole-2-carboxamide (10) (invention)

L'ortho-fluoroaniline (1.91 ml, 19.77 mmol) est mélangé à une solution de THF anhydre (13 ml) à -10°C, puis une solution de sodium bis(triméthylsilyl)amide (NaHMDS) (45.2 ml, 45,2 mmol dans du THF 1M) est introduite. Le mélange est laissé sous agitation pendant 1h à -10°C ; une suspension de **9** (1.77 g, 9.4 mmol) dans du THF anhydre (20 ml) est ensuite ajoutée, le mélange obtenu est laissé sous agitation à température ambiante, pendant 3h environ. Pour stopper la réaction, une solution d'acide acétique est introduite goutte à goutte. Il se forme alors un précipité rouge brique. Le solvant est évaporé sous vide, puis addition sur le résidu sec, d'eau et d'une solution aqueuse saturée de bicarbonate de sodium. Apparaît alors un précipité rouge-marron récupéré par filtration. Le solide obtenu est lavé à l'eau puis à l'hexane, et séché sous vide. Obtention d'un solide marron (3.24 g, 80%) ; ¹H NMR (300 MHz, DMSOd₆) δ : 8.12 (dd, 1H, ArH), 7.92 (dd, 1H, ArH), 6.92 (t, 1H, ArH), 7.13 (t, 1H, ArH), 7.07 (s, 2H, CH-CH). Analyse calculée pour C₁₀H₈FN₃O : C, 58.54 ; H, 3.93 ; N, 20.48. Expérimental : C, 58.33 ; H, 3.55 ; N, 20.14.

### 5H-Imidazo[1,2-a]quinoxalin-4-one (11) (invention) :

**10** (2 g, 9.75 mmol) est solubilisé dans du N,N-diméthylacétamide (DMA) (80 ml) avant d'ajouter de l'hydrure de sodium (NaH 60% en poids) (1.5 g, 62 mmol). Le mélange ainsi obtenu est porté à reflux pendant 15h. La réaction est suivie par CCM, éluant : MeOH/CH₂Cl₂ (10/90). Celle-ci n'étant pas complète, 2 équivalents de NaH sont ajoutés. Le mélange est laissé à reflux pendant 30h. Une fois la réaction terminée, le mélange est concentré sous vide, puis addition sur le résidu sec d'eau et d'une solution aqueuse saturée de chlorure d'ammonium. Un précipité marron se forme, il est collecté par filtration, lavé à l'eau et séché sous vide. Obtention d'un solide beige (1.42 g, 79%). Analyse calculée pour C₁₀H₇N₃O : C, 64.86 ; H, 3.81 ; N, 22.69. Expérimental : C, 64.44 ; H, 4.03 ; N, 22.98.

### 4-Chloroimidazo[1,2-a]quinoxaline (12) (invention) :

Le composé **11** (1.4 g, 6.4 mmol) est solubilisé dans de l'oxychlorure de phosphore (24 ml), et de la N,N diéthylaniline (3,6 ml). Le mélange réactionnel obtenu est porté à reflux pendant 2h environ. Le produit est insoluble dans le POCl₃ même après chauffage. La réaction est suivie par CCM, éluant : CH₂Cl₂/MeOH (95/5). Une CCM montre la présence du produit final, mais aussi l'apparition de produits de dégradation. Le mélange réactionnel est de couleur marron foncé. Le POCl₃ est évaporé sous vide. Le résidu est refroidi dans un bain de glace, avant d'ajouter un peu d'eau et, goutte à goutte, une solution saturée de bicarbonate de sodium pour neutraliser le POCl₃. Apparition d'une légère mousse jaune et d'un précipité marron. Le solide est isolé par filtration et recristallisé dans du méthanol. Obtention d'un solide beige (0.97 g, 75%) ; ¹H NMR (300 MHz, DMSOd₆) δ : 8.5 (s, 1H, N-CH-C), 8.06 (d, 1H, ArH), 7.56 (s, 1H, C-CH-N), 7.35 (m, 1H, ArH), 7.25 (m, 2H, ArH). Analyse calculée pour C₁₀H₆N₃Cl: C, 58.98 ; H, 2.97 ; N, 20.64. Expérimental : C, 59.12; H, 2.76; N, 20.45.

### N-Méthylimidazo[1,2-a]quinoxalin-4-amine (13) (invention) :

Le composé **12** (0.110 g, 0,54 mmol) est solubilisé dans EtOH (10 ml), puis une solution de méthylamine dans l'eau (40% en poids) (0.15 ml, 1.74 mmol) est introduite. Le mélange est laissé sous agitation dans une autoclave, à température ambiante pendant au moins 15h. Le produit, d'abord insoluble, se solubilise peu après. L'évolution de la réaction est contrôlée par CCM, éluant : CH₂Cl₂/MeOH (95/5). Au bout de 15h, 1.5 éq. (0,06 ml) de méthylamine sont additionnées. Après 5h d'agitation, la réaction n'évolue plus. Le solvant est évaporé sous vide à sec. Le résidu jaunâtre obtenu est solubilisé dans du dichlorométhane (10 ml). Cette phase organique est lavée à l'aide d'une solution saturée de bicarbonate de sodium (10 ml) puis de l'eau (10 ml). Elle est ensuite séchée sur Na₂SO₄ puis évaporée sous vide. Récupération d'un solide blanc. Le résidu est purifié sur colonne de gel de silice afin d'éliminer les traces de produit de départ, éluant : CH₂Cl₂/AcEt (70/30). Obtention d'un solide blanc (0.99 g, 93%) ; ¹H NMR (300 MHz, DMSO-d₆) δ : 7.92 (s, 1H, C-CH-N), 7.75 (dd, *J₁*=*1.22Hz*, *J₂*=*8.17Hz*, 1H, ArH), 7,65 (dd, *J₁*=*1.26Hz*, *J₂*=*8.06Hz,* 1H*,* ArH), 7.52 (s, 1H, N-CH-C), 7.4 (t, 1H, ArH), 7.25 (t, 1H, ArH), 6.15 (s, 1H, NH), 3.25 (d, 3H, -CH₃) ; ¹³C NMR (200 MHz, DMSO-d₆) δ : 142.25, 139.17, 131.99, 129.40, 128.17, 127.20, 125.14, 124.34, 114.14, 113.89, 29.20. Analyse calculée pour C₁₁H₁₀N₄: C, 66.65 ; H, 5.08 ; N, 28.26. Expérimental : C, 66.26 ; H, 5.53 ; N, 28.23.

### 1-Bromo-N-méthylimidazo[1,2-a]quinoxalin-4-amine (14) (invention)

Une solution de **13** (1.5g, 7.5mmol) et de N-bromosuccinimide (1.5g, 7.5mmol) dans du chloroforme est chauffée à reflux pendant 2h. Le mélange réactionnel résultant est refroidi, lavé avec une solution d'hydrogénocarbonate de sodium à 5%, séché avec le sulfate de sodium, et évaporé sous vide. Le résidu est purifié sur colonne de gel de silice, éluant : CH₂Cl₂/MeOH (85/15). Obtention d'un solide blanc (1.18 g, 57%) ; ¹H NMR (300 MHz, DMSO-d₆) δ : 9.10 (s, 1H, C-CH-N), 7.75 (dd, 1H, ArH), 7,65 (dd, 1H, ArH), 7.4 (t, 1H, ArH), 7.25 (t, 1H, ArH), 6.15 (s, 1H, NH), 3.25 (d, 3H, -CH₃). Analyse calculée pour C₁₁H₉N₄Br : C, 47.68 ; H, 3.27 ; N, 20.22. Expérimental : C, 47.33 ; H, 3.53 ; N, 20.53.

### Procédure générale de la réaction de Suzuki (invention) :

Pour un mélange de **14** (300 mg, 1,08 mmol) et de tétrakis (63 mg, 0,05 mmol) dans du DME (15 mL) est ajouté le correspondant aryl acide boronique suivie par l'ajout de carbonate de sodium (234 mg) dans de l'eau (5 mL). La réaction est irradiée sous micro-onde dans un tube scellé à 140 ° C pendant 20 min dans un synthétiseur Biotage. La réaction est versée sur de l'eau et est extraite par le dichlorométhane (2 x 40 ml). Les phases organiques sont lavées avec de l'eau (40 mL), séchées et concentrées à sec sous vide. Le produit brut est purifié par chromatographie sur colonne (gel de silice en utilisant le dichlorométhane comme éluant).

### N-Méthyl-1-phénylimidazo[1,2-a]quinoxalin-4-amine (15a) (EAPB0403) (invention)

Acide phénylboronique (260 mg, 2.13 mmol). Solide blanc (90%) ; ¹H NMR (300 MHz, CDCl₃-d₆) δ : 8.15 (s, 1H), 7.99 (dd, 1H), 7,52 (m, 8H), 5.7(s, 1H), 3.01 (d, 3H). Analyse calculée pour C₁₇H₁₄N₄ : C, 74.43 ; H, 5.14 ; N, 20.42. Expérimental : C, 74.15 ; H, 5.46 ; N, 20.09.

### 1-(3-méthoxyphényl)-N-méthylimidazo[1,2-a]quinoxalin-4-amine (15b) (EAPB0503) (invention)

Acide 3-méthoxyphénylboronique (329 mg, 2.16 mmol). Solide blanc (90%) ; ¹H NMR (300 MHz, CDCl₃-d₆) δ : 9.04 (s, 1H), 8 (dd, 1H), 7,82 (m, 2H), 7.4 (m, 3H), 6.99 (d, 1H), 6.86 (s, 1H), 5.7 (s, 1H), 3.83 (s, 3 H), 2.88 (d, 3H). Analyse calculée pour C₁₈H₁₆N₄O : C, 71.04 ; H, 5.30 ; N, 18.41. Expérimental : C, 71.40 ; H, 5.66 ; N, 18.09.

### 1-(4-méthoxyphenyl)-N-méthylimidazo[1,2-a]quinoxalin-4-amine (15c) (EAPB0703) (invention)

Acide 4-méthoxyphénylboronique (329 mg, 2.16 mmol). Solide blanc (95%). ¹H NMR (300 MHz, CDCl₃-d₆) δ : 8.14 (s, 1H), 8.00 (d, 1H), 7.67 (m, 3H), 7.5 (m, 2H), 7.05 (m, 2H), 5.77 (s, 1H), 3.82 (s, 3H), 2.99 (s, 3H). Analyse calculée pour C₁₈H₁₆N₄O: C, 71.04 ; H, 5.30 ; N, 18.41. Expérimental : C, 71.18 ; H, 5.58 ; N, 18.13.

### 1-(2-méthoxyphényl)-N-méthylimidazo[1,2-a]quinoxalin-4-amine (15d) (EAPB0803) (invention)

Acide 2-méthoxyphénylboronique (329 mg, 2.16 mmol). Solide beige (94%). ¹H NMR (300 MHz, CDCl₃-d₆) δ : 8.1 (d, 1H), 7.60 (m, 3H), 7.48 (t, 1H), 7.30 (m, 4H), 5.74 (s, 1H), 3.85 (s, 3H), 3.00 (s, 3H). Analyse calculée pour C₁₈H₁₆N₄O : C, 71.04 ; H, 5.30 ; N, 18.41. Expérimental : C, 70.98 ; H, 5.12 ; N, 18.22.

### 1-(3-éthoxyphényl)-N-méthylimidazo[1,2-a]quinoxalin-4-amine (15e) (EAPB0903) (invention)

Acide 3-éthoxyphénylboronique (360 mg, 2.17 mmol). Solide blanc (52%). ¹H NMR (300 MHz, CDCl₃-d₆) δ : 8.34 (s, 1H), 8.02 (d, 1H), 7.83 (d, 1H), 7.67 (t, 1H), 7.5 (m, 2H), 6.96 (d, 1H), 6.85 (s, 1H), 5.74 (s, 1H), 3.89 (m, 2H), 2.89 (s, 3H), 1.38 (t, 3H). Analyse calculée pour C₁₉H₁₇N₄O : C, 71.92 ; H, 5.36 ; N, 17.67. Expérimental : C, 72.13 ; H, 5.56 ; N, 17.59.

### 1-(3-hydroxyphényl)-N-méthylimidazo[1,2-a]quinoxalin-4-amine (15f) (EAPB0603) (invention)

Acide 3-hydroxyphénylboronique (329 mg, 2.16 mmol). Solide blanc (85%). ¹H NMR (300 MHz, CDCl₃-d₆) δ : 8.33 (s, 1H), 7.99 (d, 1H), 7.79 (d, 1H), 7.70 (m, 2H), 7.67 (t, 1H), 7.50 (m, 3H), 6.857 (m, 2H), 3.02 (s, 3H). Analyse calculée pour C₁₇H₁₅N₄O: C, 70.09 ; H, 5.19 ; N, 19.23. Found : C, 70.49 ; H, 5.28 ; N, 19.09.

### 1-(3-bromophényl)-N-méthylimidazo[1,2-a]quinoxalin-4-amine (15g) (EAPB01003) (invention)

Acide 3-bromophénylboronique (435 mg, 2.16 mmol). Solide jaune (78%). ¹H NMR (300 MHz, CDCl₃-d₆) δ : 8.18 (s, 1H), 7.90 (m, 2H), 7.67 (t, 1H), 7.50 (m, 2H), 7.45 (m, 3H), 5.74 (s, 1H), 2.98 (s, 3H). Analyse calculée pour C₁₇H₁₃ Br N₄ : C, 57.81 ; H, 3.71 ; N, 15.86. Expérimental : C, 58.05; H, 3.55 ; N, 15.99.

### 1-(3-(trifluorométhyl)-phényl))-N-méthylimidazo[1,2-a]quinoxalin-4-amine (15h) (EAPB01103) (invention)

Acide 3-(trifluoromethyl)-phénylboronique (411 mg, 2.16 mmol). Solide blanc (98%). ¹H NMR (300 MHz, CDCl₃-d₆) δ : 8.14 (s, 1H), 8.10 (s, 1H), 8.02 (m, 2H), 7.85 (d, 1H), 7.60 (m, 2H), 7.48 (t, 1H), 5.74 (s, 1H), 2.96 (s, 3H). Analyse calculée pour C₁₈H₁₂N₄F₃ : C, 63.34 ; H, 3.52 ; N, 16.42. Expérimental: C, 63.03 ; H, 3.86 ; N, 16.74.

### 1-(3-chlorophényl)-N-méthylimidazo[1,2-a]quinoxalin-4-amine (15i) (EAPB01203) (invention)

Acide 3-chlorophénylboronique (339 mg, 2.16 mmol). Solide jaune (63%). ¹H NMR (300 MHz, CDCl₃-d₆) δ : 8.48 (s, 1H), 8.02 (d, 1H), 7.67 (t, 1H), 7.58 (m, 2H), 7.49 (m, 3H), 7.10 (s, 1H), 5.74 (s, 1H), 2.81 (s, 3H). Analyse calculée pour C₁₇H₁₃ClN₄: C, 66.13 ; H, 4.24; N, 11.48. Expérimental : C, 66.35 ; H, 4.01 ; N, 11.25.

### 1-(3-carboxyphényl)-N-méthylimidazo[1,2-a]quinoxalin-4-amine (15j) (EAPB01303) (invention)

Acide 3-carboxyphénylboronique (357 mg, 2.16 mmol). Solide orange (87%). ¹H NMR (300 MHz, CDCl₃-d₆) δ : 8.41 (d, 1H), 8.26 (s, 1H), 8.01 (m, 2H), 7.80 (m, 4H), 7.58 (m, 2H), 7.47 (t, 1H), 2.95 (s, 3H). Analyse calculée pour C₁₈H₁₄N₄O₂ : C, 67.92 ; H, 4.43 ; N, 17.60. Expérimental : C, 67.72 ; H, 4.35; N, 17.63.

### 1-(3-fluorophényl)-N-méthylimidazo[1,2-a]quinoxalin-4-amine (15k) (EAPB01403) (invention)

Acide 3-fluorophénylboronique (303 mg, 2.16 mmol). Solide jaune (66%). ¹H NMR (300 MHz, CDCl₃-d₆) δ : 8.22 (s, 1H), 7.98 (d, 1H), 7.59 (m, 3H), 7.48 (m, 2H), 7.22 (s, 1H), 7.03 (d, 1H), 5.74 (s, 1H), 2.96 (s, 1H). Analyse calculée pour C₁₇H₁₃FN₄ : C, 69.85 ; H, 4.48 ; N, 19.17. Expérimental : C, 69.79 ; H, 4.48 ; N, 18.97.

### 1-(3-cyanophényl)-N-méthylimidazo[1,2-a]quinoxalin-4-amine (15l) (EAPB01503) (invention)

Acide 3-cyanophénylboronique (351 mg, 2.16 mmol). Solide beige (84%). ¹H NMR (300 MHz, CDCl₃-d₆) δ : 8.41 (s, 1H), 8.13 (d, 1H), 8.00 (d, 1H), 7.77 (d, 1H), 7.64 (m, 3H), 7.51 (m, 2H), 5.77 (s, 1H), 2.76 (s, 3H). Analyse calculée pour C₁₈H₁₃N₅ : C, 72.23 ; H, 4.38 ; N, 23.40. Expérimental : C, 72.01 ; H, 4.71 ; N, 23.25.

### 1-(3-nitrophényl)-N-méthylimidazo[1,2-a]quinoxalin-4-amine (15m) (EAPB01603) (invention)

Acide 3-nitrophénylboronique (399 mg, 2.16 mmol). Solide beige (95%). ¹H NMR (300 MHz, CDCl₃-d₆) δ : 8.21 (s, 1H), 8.01 (d, 1H), 7.70 (m, 2H), 7.58 (d, 1H), 7.40 (m, 2H), 7.17 (m, 2H), 5.17 (s, 1H), 3.01 (s, 3H). Analyse calculée pour C₁₇H₁₃N₅O : C, 63.94 ; H, 4.10; N, 31.93. Expérimental : C, 64.13 ; H, 3.89 ; N, 21.66.

### 1-furan-N-méthylimidazo[1,2-a]quinoxalin-4-amine (15n) (EAPB01703) (référence)

Acide furanboronique (243 mg, 2.16 mmol). Solide blanc (96). ¹H NMR (300 MHz, CDCl₃-d₆) δ : 8.24 (s, 1H), 7.90 (m, 3H), 7.83 (s, 1H), 7.62 (t, 1H), 7.50 (m, 2H), 7.12 (m, 2H), 6.98 (s, 1H), 5.74 (s, 1H), 4.85 (d, 1H), 2.85 (s, 3H). Analyse calculée pour C₁₅H₁₆N₄O : C, 68.17 ; H, 4.58 ; N, 21.20. Expérimental: C, 68.20 ; H, 4.56 ; N, 21.17.

### 2) Etudes sur le mélanome

### Etude de cytotoxicité in vitro

L'activité antiproliférative a été mesurée d'abord sur une lignée de cellules cancéreuses d'origine humaine, les A375 du mélanome. Par la suite, des tests de cytotoxicité ont été réalisés pour les molécules les plus actives sur d'autres lignées cancéreuses du mélanome mais aussi sur des lignées cancéreuses du colon, du sein, de l'ovaire et du lymphome B.

L'imiquimod et la fotémustine (Muphoran®) serviront de témoins dans cette étude. Plusieurs lignées cellulaires de carcinome humain ont été utilisées :
- Les lignées A375, M4Be, RPMI7591 issues d'un mélanome humain
- La lignée LS174T est issue d'un carcinome du colon.
- La lignée A 2780 est issu d'un carcinome ovarien.
- La lignée Raji est issue d'un lymphome B.
- La lignée MCF7 est issue d'un carcinome du sein.

L'IC₅₀ (concentration de produit testé qui inhibe 50% de la prolifération cellulaire maximale relevée dans les puits témoins) est déterminée graphiquement à partir de la courbe donnant le pourcentage de prolifération cellulaire en fonction de la concentration de produit testé.

### a) Résultats des études de cytotoxicité in vitro

Les IC₅₀ et les écarts types de chaque produit testé *in vitro* sur la lignée A375 du mélanome sont représentés dans le tableau 1.

Les composés testés ont la structure suivante :

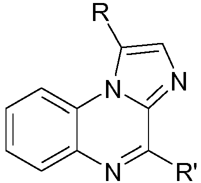

**Tableau 1 (référence): Structures des composés et leur activité cytotoxique in vitro.**

| **Composés** | **R** | **R'** | **IC₅₀ (µM)** |
|---|---|---|---|
| **EAPB0103** | (CH₃)₂-CH-CH₂- | CH₃-NH- | 31,6 ± 2,0 |
| **EAPB0203** | C₆H₅-(CH₂)₂- | CH₃-NH- | 1,57 ± 0,56 |
| **EAPB0202** | C₆H₅-(CH₂)₂- | NH₂- | 2,35 ± 0,15 |
| **EAPB0201** | C₆H₅-(CH₂)₂- | Cl- | 24,0 ± 0,5 |
| **EAPB0104** | (CH₃)₂-CH-CH₂- | (CH₃)₂-N- | 66,3 ± 7,5 |
| **EAPB0204** | C₆H₅-(CH₂)₂- | (CH₃)₂-N- | 80,1 ± 7,0 |
| **EAPB0206** | C₆H₅-(CH₂)₂- | CH₃-O- | 47,8 ± 5,0 |

On constate d'après la comparaison des IC₅₀ que l'on a identifié deux molécules très actives le **EAPB0203** (IC₅₀=1,57 µM) et le **EAPB0202** (IC₅₀=2,35 µM) parmi les composés testés. En effet, le **EAPB0203** qui correspond à notre composé « lead » a une activité 110 fois supérieure à celle de la fotémustine et 50 fois supérieure à celle de l'imiquimod. Le **EAPB0202** présente une activité très voisine de **l'EAPB0203** (quasiment 2 fois plus actif que le **EAPB0204**) avec une IC₅₀ 70 fois supérieure à la fotémustine et 30 fois supérieure à l'imiquimod. Les autres composés ont une IC₅₀ qui varie entre 20 et 100 µM. Les IC₅₀ de la fotémustine et de l'imiquimod sont présentés dans le tableau 2.

Ensuite notre composé « lead » **l'EAPB0203** a été testé sur d'autre lignées cellulaire du mélanome humain (M4Be et RPMI7591), les LS174T (cancer du colon), les MCF7 (cancer du sein), et les Raji (lymphome B) pour observer son éventuelle activité en le comparant à l'imiquimod et aux molécules de référence convenables pour chaque type de cancer.

Les IC₅₀ sont illustrées dans le tableau 2.

**Tableau 2 (référence): Les IC₅₀ de l'imiquimod, fotemustine, methothrexate, irinotecan, doxorubicin et EAPB0203 sur les A35, M4Be et RMPI 7590 (mélanome), LS174T (cancer du colon), MCF7 (cancer du sein), Raji (lymphome B).**

| **composés** | **IC50 (µM)** | | | | |
|---|---|---|---|---|---|
| **Raji** | **A375** | **M4Be** | **RPMI7591** | **MCF7** | **LS174T** |
| Fotemustine | 173 ± 24 | 326 ± 34 | 125 ± 49 | | |
| Doxorubicine | | | | 0.13 ± 0.01 | |
| Irinotecan | | | | | 1.16 ± 0.07 |
| Methothrexate 0.04 ± 0.005 | | | | | |
| Imiquimod 139 ± 12 | 70.3 ± 4.3 | 33.5 ± 7.7 | 53.7 ± 9.8 | 145 ± 7 | 34.4 ± 9.7 |
| **EAPB0203** 6.23 ± 0.06 | 1.57 ± 0.56 | 2.58 ± 0.40 | 4.23 ± 0.48 | 1.08 ± 0.46 | 4.12 ± 0.67 |

On conclut que l'**EAPB0203** présente des activités de l'ordre de µM sur les différentes lignées cellulaires cancéreuses testées avec une activité significative sur le mélanome par rapport à celle de la molécule de référence, la fotémustine.

L'application de la deuxième stratégie de synthèse sur la série imidazo[1,2-*a*]quinoxaline, nous a permis de synthétiser d'autres molécules (invention). Ces dernières ont été évaluées *in vitro* sur les A375. Les IC₅ₒ sont illustrées dans le tableau 3 en comparaison avec l'**EAPB0203.**

**Tableau 3 (invention): Structures des composés et leur activité cytotoxique in vitro.**

| ***Composés*** | ***R*** | ***Formules*** | ***IC₅₀ (µM)*** |
|---|---|---|---|
| **15a (EAPB0403)** | C₆H₅- | C₁₈H₁₆N₄O | 2,19 ± 0.08 |
| **15b (EAPB0503)** | 3-OCH₃-C₆H₅₋ | C₁₈H₁₆N₄O | 0.18 ± 0.09 |
| **15c (EAPB0703)** | 4-OCH₃-C₆H₅₋ | C₁₈H₁₆N₄O | 0.37 ± 0.4 |
| **15d (EAPB0803)** | 2-OCH₃-C₆H₅₋ | C₁₈H₁₆N₄O | 122 ± 25 |
| **15e (EAPB0903)** | 3-OC₂H₅-C₆H₅₋ | C₁₉H₁₇N₄O | 0.30 ± 0.01 |
| **15f (EAPB0603)** | 3-OH-C₆H₅₋ | C₁₇H₁₅N₄O | 0.56 ± 0.12 |
| **15g (EAPB01003)** | 3-Br-C₆H₅₋ | C₁₇H₁₃ BrN₄ | 0.65 ± 0.02 |
| **15h (EAPB01103)** | 3-CF₃-C₆H₅₋ | C₁₈H₁₂N₄F₃ | 1.28 ± 0.18 |
| **15i (EAPB01203)** | 3-Cl-C₆H₅- | C₁₇H₁₃ClN₄ | 1.78 ± 0.35 |
| **15j (EAPB01303)** | 3-COOH-C₆H₅₋ | C₁₈H₁₄N₄O₂ | 3.47 ± 0.55 |
| **15k (EAPB01403)** | 3-F-C₆H₅₋ | C₁₇H₁₃ FN₄ | 24.9 ± 0.27 |
| **15l (EAPB01503)** | 3-CN-C₆H₅₋ | C₁₈H₁₃N₅ | 27.0 ± 2.2 |
| **15m (EAPB01603)** | 3-NO₂-C₆H₅₋ | C₁₇H₁₃N₅O | 40.0 ± 3.9 |
| **15n (EAPB01703) (référence)** | (C₄H₃O)- | C₁₅H₁₆N₄O | 74.1 ± 3.1 |
| **EAPB0203 (référence)** | C₆H₅-(CH₂)₂- | C₁₉H₁₈N₄ | 1.57 ± 5.6 |

La substitution de **EAPB0203** par différents groupements aryles en position R et par une méthylamine en position R' (Tableau 3) nous a permis d'obtenir une librairie de composés. En comparant les IC₅₀ on trouve que certains de ces composés prometteurs (**15b, 15c, 15e, 15f, 15g**) présentent des activités supérieures à celle de **EAPB0203.** En outre, tous les composés testés présentent une activité supérieure à celle de la fotémustine et de l'imiquimod, utilisées comme références.

Très récemment, de nouveaux composés présentant la substitution R en position 2 au lieu de la position 1 sur le cycle imidazole comme pour les composés précédents, apporteraient une nette amélioration de l'activité anticancéreuse sur le mélanome (A375). En effet, par exemple, le composé correspondant au **EAPB0503** avec la substitution 3-methoxyphényl en position 2 présente une activité 10 fois supérieure (IC₅₀ = 0,018µM) à celle de **EAPB0503** et 100 fois supérieure à **EAPB0203.**

### Etude de l'activité anti-proliférative de l'EAPB0203 in vivo (mélanome) (référence)

Dans cette étude, nous présentons des données relatives à l'activité de l'**EAPB0203** sur le volume d'un type de modèle tumoral du mélanome humain (lignées M4Be), xénogreffés à des souris athymiques. Cette étude a nécessité au préalable plusieurs essais de greffes de lignées cellulaires du mélanome humain sur souris athymiques avant d'établir le modèle animal adéquat. L'étude *in vivo* fait suite aux essais *in vitro* décrit précédemment.

### a) Détermination de la dos léthale 50 (DL₅₀) de l'EAB0203 chez la souris

La DL₅₀ de l'**EAPB0203** a été déterminée en utilisant trois doses 30mg/kg, 300mg/kg, et 450mg/kg. Après 48h, **l'EAPB0203** aux doses les plus élevées n'a entraîné aucune toxicité apparente. Elle ne montre donc aucune toxicité aigue (DL₅₀ > 450 mg/kg) chez la souris.

### b) Activité de l'EAPB0203 in vivo

L'**EAPB0203** induit une cytotoxicité significative *in vitro* sur les cellules du mélanome humain et notamment les M4Be. L'activité et la spécificité de l'**EAPB0203** *in vivo* sur la croissance tumorale du mélanome humain sont évaluées par un suivi de l'évolution tumorale chez des souris porteuses d'un mélanome M4Be traitées par administration de fotémustine et de **EAPB0203** à différentes doses.
Pour cette étude, 18 souris SWISS nude (3 groupes de six souris) ont été utilisées. Des cellules cancéreuses M4Be mises en suspension ont été injectées en sous-cutané dans le flanc droit de chaque souris SWISS athymique nude femelle âgée de 7 semaines et pesant de 20-22g au début du protocole. Les souris ont ensuite été maintenues sous atmosphère stérile et ont reçu de la nourriture et de l'eau aseptiques. Le poids de la souris ainsi que la croissance de la tumeur dans les trois dimensions ont été controlés de façon bi-hebdomadaire.
Un premier groupe de 6 souris (référence) recevra la fotémustine en intra-péritonéal à la dose de 20mg/kg une fois par semaine pendant 3 semaines, en débutant 7 jours après l'inoculation au moment où la tumeur sera visible et palpable. Un deuxième groupe de 6 souris recevra l'**EAPB0203** à la même dose de 20 mg/Kg en intra-péritonéal mais deux fois par semaine (soit 40 mg/Kg par semaine). Le troisième groupe (témoin) de 6 souris recevra seulement le véhicule d'administration. Le volume tumoral est calculé par la formule suivante : longueur x largeur x hauteur x 0.52, et est exprimé en moyenne ± SEM mm³.
Pour la fotémustine et **EAPB0203,** le rythme des administrations est d'une cure de trois semaines, arrêt de deux semaines, et une seconde cure de trois semaines.

**EAPB0203** (20 mg/kg deux fois par semaine) a été bien toléré sans pertes de poids ou effets secondaires apparents. Les souris traitées par **EAPB0203** ont présenté un retard significatif de la croissance tumorale par rapport à celles des souris contrôles et des souris fotémustine (Figure 1). Dans le groupe contrôle, les dernières souris ont été sacrifiées à *J55*, le volume tumoral ayant atteint 2 cm³. Dans le groupe fotémustine les dernières souris ont été euthanasiées à *J76* alors que dans le groupe **EAPB0203** une souris présentait encore un volume tumoral inférieur à 2 cm³.

### c) Etude pharmacocinétique

Des méthodes de dosage des composés **EAPB0203 (référence), EAPB0503** et **EAPB0603 (invention)** par chromatographie liquide haute performance et détection par spectrométrie de masse (LC/ESI-MS) ont été développées et validées dans les plasmas humains et de rat. Le prétraitement des échantillons consiste en une extraction solide liquide précédée d'une précipitation des protéines plasmatiques en milieu acide. La colonne chromatographique utilisée est une C8 Zorbax eclipse XDB et la phase mobile (gradient) un mélange d'acétonitrile et de tampon formiate (pH 3) (débit, 0.8 mL/min). La fidélité des méthodes développées est ≤ 14% et l'exactitude varie de 92 à 113%. Les coefficients d'extraction sont supérieurs à 72%. La limite de quantification est de 5 µg/l pour tous les analytes. Des essais de stabilité des composés dans les matrices ont également été réalisés. Ces méthodes ont été utilisées pour déterminer les paramètres pharmacocinétiques des composés **EAPB0203, EAPB0503** et **EAPB0603** chez le rat. Les doses létales 50% des composés **EAPB0203** et **EAPB0503** sont respectivement 14,8 et 7,6 mg/kg. Chez le rat, le composé **EAPB0603** est le métabolite actif de l'**EAPB0503.** Après administration intraveineuse de l'**EAPB0503** à la dose de 5 mg/kg, les paramètres pharmacocinétiques sont les suivants: i) **EAPB0503:** clairance totale, 2,2 L/h/kg; volume de distribution à l'équilibre, 5,6 L/kg; aire sous la courbe des concentrations plasmatiques, 2,31 mgxh/L et demi-vie d'élimination, 1,76 h: ii) **EAPB0603:** aire sous la courbe des concentrations plasmatiques, 0,439 mgxh/L et demi-vie d'élimination, 4,7 h ; et **EAPB0203** : clairance totale, 2,9 L/h/kg; volume de distribution à l'équilibre, 10,6 L/kg; aire sous la courbe des concentrations plasmatiques, 0,87 mgxh/L et demi-vie d'élimination, 2.6 h.

### d) Toxicité semichronique des composés EAPB0503 (invention) et EAPB0203 (référence) chez le rat

Les animaux ont été répartis au hasard en quatre groupes :
- groupe 1 : 5 animaux reçoivent EAPB0203 par voie intraveineuse à la dose de 5 mg/kg, 1 fois par jour pendant 5 jours,
- groupe 2 : 5 animaux reçoivent EAPB0503 par voie intraveineuse à la dose de 3 mg/kg, 1 fois par jour pendant 5 jours,
- groupe 3 : 3 animaux reçoivent le véhicule (100 µl DMSO) 1 fois par jour pendant 5 jours,
- groupe 4 : 3 animaux sont non traités et servent de témoin.

Les composés étudiés étant insoluble en milieu aqueux, ces derniers sont solubilisés dans 100 µl de DMSO puis administrés dans la veine de la queue.

***Suivi Clinique*** : un examen clinique de chaque rat est effectué deux fois par jour afin de noter tous signes de toxicité ou de changement de comportement. Le poids est contrôlé chaque jour ainsi que la consommation d'eau et de nourriture.

***Tests hématologiques*** : afin d'évaluer une possible toxicité hématologique des composés étudiés, 0.5 ml de sang a été prélevé par ponction cardiaque sur tubes contenant de l'EDTA, avant le début du traitement puis 4 et 7 jours après. Les tests hématologiques suivants ont été effectués :
- numération des globules rouges
- numération des plaquettes,
- numération des globules blancs,
- taux d'hémoglobine,
- hématocrite.

Après 7 jours, les animaux sont euthanasiés au sevoflurane et un examen macroscopique des principaux organes (foie, rein, rate, poumons, coeur et cerveau) est réalisé. Les organes ont ensuite été prélevés en vue d'un examen anatomopathologique.

Le traitement a été bien toléré par tous les animaux. La croissance pondérale ainsi que la consommation d'eau et de nourriture ont été comparables entre animaux traités et animaux témoins. Aucune toxicité hématologique n'a été observée. L'examen macroscopique des organes n'a révélé aucune anomalie ; l'examen anatomopathologique est en cours.

### 3) Etudes sur les lymphomes à cellules T (référence)

L'activité *in vitro* de **EAPB0203** sur des cellules de leucémie T de l'adulte (ATL) transformées par le rétrovirus HTLV-I (HuT-102, MT2..) et des cellules T malignes HTLV-I-négatives (CEM, Jurkatt...) a été évaluée. Dans un premier temps, l'effet de **EAPB0203** sur la croissance cellulaire de ces lignées a été évalué par deux techniques, le kit non radioactif de prolifération cellulaire 'CellTiter 96®' et la méthode d'exclusion au bleu trypan. Les concentrations de **EAPB0203,** de 1 à 10 µM, ont eu comme conséquence une inhibition progressive et dose dépendante de la croissance des cellules d'ATL et des cellules T malignes non associées à HTLV-I. **EAPB0203** a également induit une inhibition de la prolifération cellulaire des cellules leucémiques fraîches dérivées de deux patients atteints d'ATL. En revanche les lymphocytes normaux activés ou non par la PHA et provenant de deux sujets sains ont été complètement résistants à **EAPB0203** (Figure 2).
Les mécanismes d'inhibition de la prolifération cellulaire provoquée par **EAPB0203** dans les lignées cellulaires étudiées ont été déterminés par l'analyse par cytométrie en flux (CMF). Cette étude a permis d'évaluer la distribution des cellules dans les différentes phases du cycle cellulaire. Pour les cellules traitées, une accumulation progressive des cellules en phase G2 a été observée alors que le nombre de cellules décroît en phase G1. L'analyse du cycle cellulaire met aussi en évidence la présence d'une population apoptotique pré-G0/G1 dans les différentes lignées cellulaires traitées. Ce pic pré-G0/G1 correspond à des cellules dont le contenu en ADN est diminué par perte de fragments d'ADN clivés au cours de la mort cellulaire (Figure 3).

La détermination du mécanisme d'action a été étudiée en partant de l'hypothèse que l'imiquimod induit la mort cellulaire par apoptose via la voie intrinsèque mitochondriale. Une étude de l'apoptose par un double marquage membranaire à l'Annexin-V conjuguée au FITC et nucléaire à l'iodure de propidium (PI) suivie d'une analyse par microscopie à fluorescence a été réalisée. Les cellules apoptotiques montrant l'externalisation de la phosphatidylsérine (PS) ont été marquées par l'AnnexinV+ mais restent PI-, tandis que les cellules mortes sont PI+. L'analyse par microscopie à fluorescence a permis de déterminer le pourcentage de cellules en apoptose après traitement. Une expérience semblable a été également exécutée avec le marquage de la chromatine au Hoechst 33342. Ceci permet l'évaluation du pourcentage de la condensation de la chromatine, qui est un indice d'apoptose.

La mitochondrie joue un rôle central dans les mécanismes apoptotiques. La première perturbation cellulaire détectable au cours du processus apoptotique est une diminution du potentiel transmembranaire mitochondrial.

Cette diminution a été mise en évidence à l'aide d'un marquage à la Rhodamine 123 suivi d'une analyse par CMF sur les cellules en suspension.

La localisation sub-cellulaire du cytochrome c dans les lignées cellulaires traitées révèle la présence du cytochrome c dans le cytoplasme après traitement par **EAPB0203,** indiquant un relargage du cytochrome c par les mitochondries au cours de l'apoptose.
L'apoptose observée est étudiée afin de savoir si elle est due ou non à l'activation des caspases. Des extraits protéiques cellulaires ont été obtenus après divers traitements et l'évaluation de l'expression de certaines protéines a été étudiée par western blot. En effet, dans les cellules CEM et HuT-102, l'apoptose induite par **EAPB0203** a été associée à l'activation des caspases, comme le montre le clivage du PARP, et des procaspase-3, procaspase-8, et procaspase -9 en leurs formes activées. De plus, le co-traitement par l'inhibiteur des caspases zVAD a permis une protection partielle de l'apoptose induite par **EAPB0203,** ce qui constitue une démonstration directe de l'implication des caspases dans la mort cellulaire et l'inhibition de la croissance induites par

### EAPB0203.

L'activation de la voie mitochondriale est régulée par les membres de la famille Bcl-2. **EAPB0203** a induit une diminution significative de l'expression des protéines IAP-1 (inhibiteurs des caspases) et bcl-xL (protéine anti-apoptotique). Dans les lignées cellulaires infectées ou non par le virus HTLV-I, l'effet de **EAPB0203** sur les régulateurs du cycle cellulaire est étudié. Une augmentation significative de l'expression des protéines p21 et p53 est observée dans les cellules HuT-102 et MT2 (HTLV-I-positives) traitées avec **EAPB0203.**

Ces résultats laissent supposer que p53 joue un rôle important dans l'arrêt du cycle cellulaire en G₂. En effet, p53 régule positivement la transcription de p21. L'activation de p21 par p53 provoque une diminution d'expression de la cycline B et de Cdc2 et par la suite l'arrêt du cycle cellulaire en G₂/M.
L'implication de l'apoptose après traitement par **EAPB0203** est mise en évidence. En effet, Une activation de la voie intrinsèque mitochondriale dans les cellules étudiées est démontrée. La chute du potentiel transmembranaire mitochondrial obtenu après traitement est en faveur d'un processus pro-apoptotique permettant l'ouverture des pores mitochondriaux et donc la libération des molécules apoptogènes comme le cytochrome c.

L'étude de l'effet de **EAPB0203,** sur la prolifération cellulaire et sur l'apoptose des cellules T malignes transformées et des cellules HTLV-I-négatives démontre des effets sélectifs sur les cellules malignes, et soutient un rôle thérapeutique pour **EAPB0203** chez les patients présentant l'ATL et d'autres lymphomes HTLV-I-négatifs, comme thérapie systémique ou topique pour des lymphomes cutanés.

## Revendications

1. Composé pour le traitement des cancers de formule (IX) : 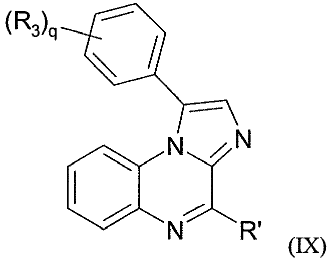 dans laquelle :
R₃ représente un atome d'hydrogène, un halogène ou un groupe choisi parmi les groupes méthyle, éthyle, propyle, isopropyle, butyle, isobutyle, tertiobutyle, -CF₃, CN, CHO, NO₂ et -(CH₂)_{n"}-(CH=CH)-(CH₂)_{n'"}-CH₃ avec n" et n'" compris indépendamment entre 0 et 4, COOR₄, NR₄R₅ et OR₄,
R₄ et R₅ représentent indépendamment un atome d'hydrogène ou groupe choisi parmi les radicaux alkyle en C₁-C₄ linéaire ou ramifié alkényle en C₁-C₄ linéaire ou ramifié, cycloalkyle en C₃-C₇
q est compris entre 1 et 5
R' représente un groupe chloro, méthoxy, amino, méthylamino, diméthylamino, éthylamino, diéthylamino, aminométhylamine ou aminoéthylamine,
et ses sels physiologiquement acceptables.

2. Composé pour le traitement des cancers selon la revendication 1 dans lequel R₃ est choisi parmi H, Cl, Br, F, hydroxy, méthyl, méthoxy, éthoxy, -CF₃, CN, COOH, COOCH₃, COOCH₂CH₃,.

3. Composé pour le traitement des cancers selon l'une des revendications précédentes dans lequel R₃ est choisi parmi H, hydroxy, méthoxy, éthoxy, Br, CF₃, Cl et COOH.

4. Composé pour le traitement des cancers selon l'une des revendications précédentes dans lequel R' est -NH-CH₃, -NH₂ ou -NH-(CH₂)₂-NH₂.

5. Composé pour le traitement des cancers selon l'une des revendications précédentes dans lequel R' est -NH-CH₃ ou -NH₂; R₃ est hydroxy ou méthoxy et q = 1.

6. Composé pour le traitement des cancers selon la revendication 1 choisi parmi les composés suivants :
*N*-Méthyl-1-phénylimidazo[1,2-*a*]quinoxalin-4-amine, *N*-méthyl-1-(2-hydroxyphényl)imidazo[1,2-*a*]quinoxalin-4-amine, *N*-méthyl-1-(3-hydroxyphényl)imidazo[1,2-*a*]quinoxalin-4-amine, *N*-méthyl-1-(4-hydroxyphényl)imidazo[1,2-*a*]quinoxalin-4-amine, *N*-Méthyl-1-(2,4-dihydroxyphényl)imidazo[1,2-*a*]quinoxalin-4-amine, *N*-Méthyl-1-(2,3-dihydroxyphényl)imidazo[1,2-*a*]quinoxalin-4-amine, *N*-Méthyl-1-(2-méthoxyphényl)imidazo[1,2-*a*]quinoxalin-4-amine, *N*-Méthyl-1-(3-méthoxyphényl)imidazo[1,2-*a*]quinoxalin-4-amine, *N*-Méthyl-1-(4-méthoxyphényl)imidazo[1,2-*a*]quinoxalin-4-amine, *N*-Méthyl-1-(2,4-diméthoxyphényl)imidazo[1,2-*a*]quinoxalin-4-amine, *N*-Méthyl-1-(2,3-diméthoxyphényl)imidazo[1,2-*a*]quinoxalin-4-amine, *N*-Méthyl-1-(3-éthoxyphényl)imidazo[1,2-*a*]quinoxalin-4-amine, *N*-Méthyl-1-(4-éthoxyphényl)imidazo[1,2-*a*]quinoxalin-4-amine, *N*-méthyl-1-(3-bromophényl)imidazo[1,2-*a*]quinoxalin-4-amine, *N-*méthyl-1-(4-bromophényl)imidazo[1,2-*a*]quinoxalin-4-amine, *N*-méthyl-1-(3-(trifluorométhyl)phényl))imidazo[1,2-*a*]quinoxalin-4-amine, *N*-méthyl-1-(4-(trifluorométhyl)phényl))imidazo[1,2-*a*]quinoxalin-4-amine, *N*-méthyl-1-(3-chlorophényl)imidazo[1,2-*a*]quinoxalin-4-amine, *N*-méthyl-1-(4-chlorophényl)imidazo[1,2-*a*]quinoxalin-4-amine, *N*-méthyl-1-(3-carboxyphényl)imidazo[1,2-*a*]quinoxalin-4-amine, *N-*méthyl-1-(4-carboxyphényl)imidazo[1,2-*a*]quinoxalin-4-amine, *N*-méthyl-1-(3-fluorophényl)imidazo[1,2-*a*]quinoxalin-4-amine, *N*-méthyl-1-(4-fluorophényl)imidazo[1,2-*a*]quinoxalin-4-amine, *N*-méthyl-1-(3-cyanophényl)imidazo[1,2-*a*]quinoxalin-4-amine, *N-*méthyl-1-(4-cyanophényl)imidazo[1,2-*a*]quinoxalin-4-amine, *N*-méthyl-1-(3-nitrophényl)imidazo[1,2-*a*]quinoxalin-4-amine, *N*-méthyl-1-(4-nitrophényl)imidazo[1,2-*a*]quinoxalin-4-amine, *N*-(2-aminoéthyl)-1-(2-hydroxyphényl)imidazo[1,2-*a*]quinoxalin-4-amine, *N*-(2-aminoéthyl)-1-(3-hydroxyphényl)imidazo[1,2-*a*]quinoxalin-4-amine, *N*-(2-aminoéthyl)-1-(4-hydroxyphényl)imidazo[1,2-*a*]quinoxalin-4-amine, *N*-(2-aminoéthyl)-1-(2,4-dihydroxyphényl)imidazo[1,2-*a*]quinoxalin-4-amine, *N*-(2-aminoéthyl)-1-(2,3-dihydroxyphényl)imidazo[1,2-*a*]quinoxalin-4-amine, *N*-(2-aminoéthyl)-1-(2-méthoxyphényl)imidazo[1,2-*a*]quinoxalin-4-amine, *N*-(2-aminoéthyl)-1-(3-méthoxyphényl)imidazo[1,2-*a*]quinoxalin-4-amine, *N*-(2-aminoéthyl)-1-(4-méthoxyphényl)imidazo[1,2-*a*]quinoxalin-4-amine, *N*-(2-aminoéthyl)-1-(2,4-diméthoxyphényl)imidazo[1,2-*a*]quinoxalin-4-amine, *N*-(2-aminoéthyl)-1-(2,3-diméthoxyphényl)imidazo[1,2-*a*]quinoxalin-4-amine, *N*-(2-aminoéthyl)-1-(3-éthoxyphényl)imidazo[1,2-*a*]quinoxalin-4-amine, *N*-(2-aminoéthyl)-1-(4-éthoxyphényl)imidazo[1,2-*a*]quinoxalin-4-amine, *N*-(2-aminoéthyl)-1-(3-bromophényl)imidazo[1,2-*a*]quinoxalin-4-amine, *N*-(2-aminoéthyl)-1-(4-bromophényl)imidazo[1,2-*a*]quinoxalin-4-amine, *N*-(2-aminoéthyl)-1-(3-(trifluorométhyl)phényl))imidazo[1,2-*a*]quinoxalin-4-amine, *N*-(2-aminoéthyl)-1-(4-(trifluorométhyl)phényl))imidazo[1,2-*a*]quinoxalin-4-amine, *N-*(2-aminoéthyl)-1-(3-chlorophényl)imidazo[1,2-*a*]quinoxalin-4-amine, *N-*(2-aminoéthyl)-1-(4-chlorophényl)imidazo[1,2-*a*]quinoxalin-4-amine, *N*-(2-aminoéthyl)-1-(3-carboxyphényl)imidazo[1,2-*a*]quinoxalin-4-amine, *N*-(2-aminoéthyl)-1-(4-carboxyphényl)imidazo[1,2-*a*]quinoxalin-4-amine, *N*-(2-aminoéthyl)-1-(3-fluorophényl)imidazo[1,2-*a*]quinoxalin-4-amine, *N*-(2-aminoéthyl)-1-(4-fluorophényl)imidazo[1,2-*a*]quinoxalin-4-amine, *N*-(2-aminoéthyl)-1-(3-cyanophényl)imidazo[1,2-*a*]quinoxalin-4-amine, *N-*(2-aminoéthyl)-1-(4-cyanophényl)imidazo[1,2-*a*]quinoxalin-4-amine, *N*-(2-aminoéthyl)-1-(3-nitrophényl)imidazo[1,2-*a*]quinoxalin-4-amine, *N*-(2-aminoéthyl)-1-(4-nitrophényl)imidazo[1,2-*a*]quinoxalin-4-amine, 1-phénylimidazo[1,2-*a*]quinoxalin-4-amine, 1-(2,4-dihydroxyphényl)imidazo[1,2-*a*]quinoxalin-4-amine, 1-(2,3-dihydroxyphényl)imidazo[1,2-*a*]quinoxalin-4-amine, 1-(2-méthoxyphényl)imidazo[1,2-*a*]quinoxalin-4-amine, 1-(3-méthoxyphényl)imidazo[1,2-*a*]quinoxalin-4-amine, 1-(4-méthoxyphényl)imidazo[1,2-*a*]quinoxalin-4-amine, 1-(2,4-diméthoxyphényl)imidazo[1,2-*a*]quinoxalin-4-amine, 1-(2,3-diméthoxyphényl)imidazo[1,2-*a*]quinoxalin-4-amine, 1-(3-éthoxyphényl)imidazo[1,2-*a*]quinoxalin-4-amine, 1-(4-éthoxyphényl)imidazo[1,2-*a*]quinoxalin-4-amine, 1-(3-bromophényl)imidazo[1,2-*a*]quinoxalin-4-amine, 1-(4-bromophényl)imidazo[1,2-*a*]quinoxalin-4-amine, 1-(3-(trifluorométhyl)phényl))imidazo[1,2-*a*]quinoxalin-4-amine, 1-(4-(trifluorométhyl)phényl))imidazo[1,2-*a*]quinoxalin-4-amine, 1-(3-chlorophényl)imidazo[1,2-*a*]quinoxalin-4-amine, 1-(4-chlorophényl)imidazo[1,2-*a*]quinoxalin-4-amine, 1-(3-carboxyphényl)imidazo[1,2-*a*]quinoxalin-4-amine, 1-(4-carboxyphényl)imidazo[1,2-*a*]quinoxalin-4-amine, 1-(3-fluorophényl)imidazo[1,2-*a*]quinoxalin-4-amine, 1-(4-fluorophényl)imidazo[1,2-*a*]quinoxalin-4-amine, 1-(3-cyanophényl)imidazo[1,2-*a*]quinoxalin-4-amine, 1-(4-cyanophényl)imidazo[1,2-*a*]quinoxalin-4-amine, 1-(3-nitrophényl)imidazo[1,2-*a*]quinoxalin-4-amine, 1-(4-nitrophényl)imidazo[1,2-*a*]quinoxalin-4-amine, 4-Chloro-1-phénylimidazo[1,2-*a*]quinoxaline, 4-Chloro-1-(2-hydroxyphényl)imidazo[1,2-*a*]quinoxaline, 4-Chloro-1-(3-hydroxyphényl)imidazo[1,2-*a*]quinoxaline, 4-Chloro-1-(4-hydroxyphényl)imidazo[1,2-*a*]quinoxaline, 4-Chloro-1-(2-méthoxyphényl)imidazo[1,2-*a*]quinoxaline, 4-Chloro-1-(3-méthoxyphényl)imidazo[1,2-*a*]quinoxaline, 4-Chloro-1-(4-méthoxyphényl)imidazo[1,2-*a*]quinoxaline, 4-Chloro-1-(2,4-diméthoxyphényl)imidazo[1,2-*a*]quinoxaline, 4-Chloro-1-(2,3-diméthoxyphényl)imidazo[1,2-*a*]quinoxaline, 4-Méthoxy-1-phénylimidazo[1,2-*a*]quinoxaline, 4-Méthoxy-1-(2-hydroxyphényl)imidazo[1,2-*a*]quinoxaline, 4-Méthoxy-1-(3-hydroxyphényl)imidazo[1,2-*a*]quinoxaline, 4-Méthoxy-1-(4-hydroxyphényl)imidazo[1,2-*a*]quinoxaline, 4-Méthoxy-1-(2-méthoxyphényl)imidazo[1,2-*a*]quinoxaline, 4-Méthoxy-1-(3-méthoxyphényl)imidazo[1,2-*a*]quinoxaline, 4-Méthoxy-1-(4-méthoxyphényl)imidazo[1,2-*a*]quinoxaline, 4-Méthoxy-1-(2,4-diméthoxyphényl)imidazo[1,2-*a*]quinoxaline, 4-Méthoxy-1-(2,3-diméthoxyphényl)imidazo[1,2-*a*]quinoxaline, et leurs sels physiologiquement acceptables.

7. Composé pour le traitement des cancers selon la revendication 1 choisi parmi les composés suivants :
*N*-méthyl-1*-*(3-hydroxyphényl)imidazo[1,2-*a*]quinoxalin-4-amine, *N*-Méthyl-1-(3-méthoxyphényl)imidazo[1,2-*a*]quinoxalin-4-amine, 1-(3-méthoxyphényl)imidazo[1,2-*a*]quinoxalin-4-amine et leurs sels physiologiquement acceptables.

8. Composé pour le traitement des cancers selon l'une des revendications précédentes **caractérisé en ce que** le cancer est choisi parmi les mélanomes et les lymphomes.

9. Composition pharmaceutique pour le traitement des cancers comprenant un composé selon l'une des revendications précédentes et un véhicule pharmaceutique approprié.

10. Produit comprenant un composé pour le traitement des cancers selon l'une des revendications 1 à 7 et un autre agent actif comme produit de combinaison pour une utilisation simultanée, séparée ou étalée dans le temps en thérapie.

## Patentansprüche

1. Verbindung zur Behandlung von Krebserkrankungen der Formel (IX): 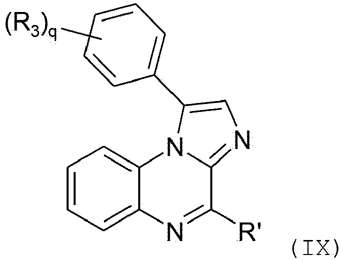 In der:
R₃ ein Wasserstoffatom, ein Halogen oder eine Gruppe darstellt, die unter der Methyl-, Ethyl-, Propyl-, Isopropyl-, Butyl-, Isobutyl-, Tertiärbutyl-Gruppe, -CF₃, CN, CHO, NO₂ und -CH_{2n"}-(CH=CH)-(CH₂)_{n'''}-CH₃ mit n" und n''' unabhängig zwischen 0 und 4, COOR₄, NR₄R₅ und OR₄ ausgewählt ist,
R₄ et R₅ unabhängig voneinander ein Wasserstoffatom oder eine Gruppe darstellen, die unter linearem oder
verzweigtem C₁-C₄-Alkyl, linearem oder verzweigtem C₁-C₄-Alkenyl, C₃-C₇-Cycloalkyl ausgewählt ist,
q zwischen 1 und 5 liegt
R' stellt eine Chlor-, Methoxy-, Amino-, Methylamino-, Dimethylamino-, Ethylamino-, Diethylamino-, Aminomethylamin- oder Aminoethylamin-Gruppe und deren physiologisch annehmbare Salze dar.

2. Verbindung zur Behandlung von Krebserkrankungen nach Anspruch 1, worin R₃ unter H, Cl, Br, F, Hydroxy, Methyl, Methoxy, Ethoxy, -CF₃, CN, COOH, COOCH₃, COOCH₂CH₃ ausgewählt ist.

3. Verbindung zur Behandlung von Krebserkrankungen nach einem beliebigen der vorstehenden Ansprüche, wobei R₃ unter H, Hydroxy, Methoxy, Ethoxy, Br, CF₃, Cl und COOH ausgewählt ist.

4. Verbindung zur Behandlung von Krebserkrankungen nach einem beliebigen der vorstehenden Ansprüche, wobei R' -NH-CH₃ ist, -NH₂ oder -NH-(CH₂)₂-NH₂.

5. Verbindung zur Behandlung von Krebserkrankungen nach einem beliebigen der vorstehenden Ansprüche, wobei R' -NH-CH₃ oder -NH₂ ist; R₃ ist Hydroxy oder Methoxy, und q = 1.

6. Verbindung zur Behandlung von Krebserkrankungen nach Anspruch 1, die unter den folgenden Verbindungen gewählt ist:
*N*-Methyl-1-Phenylimidazo[1,2-*a*]Quinoxalin-4-amin, *N-*Methyl-1-(2-Hydroxyphenyl)imidazo[1,2-*a*]Quinoxalin-4-amin, *N*-Methyl-1-(3-Hydroxyphenyl)imidazo[1,2-*a*]Quinoxalin-4-amin, *N*-Methyl-1-(4-Hydroxyphenyl)imidazo[1,2-*a*]Quinoxalin-4-amin, *N-*Methyl-1-(2,4-Dihydroxyphenyl)imidazo[1,2-*a*]Quinoxalin-4-amin, *N*-Methyl-1-(2,3-Dihydroxyphenyl)imidazo[1,2-*a*]Quinoxalin-4-amin, *N*-Methyl-1-(2-Methoxyphenyl)imidazo[1,2-*a*]Quinoxalin-4-amin, *N-*Methyl-1-(3-Methoxyphenyl)imidazo[1,2-*a*]Quinoxalin-4-amin, *N*-Methyl-1-(4-Methoxyphenyl)imidazo[1,2-*a*]Quinoxalin-4-amin, *N*-Methyl-1-(2,4-Dimethoxyphenyl)imidazo[1,2-*a*]Quinoxalin-4-amin, *N-*Methyl-1-(2,3-Dimethoxyphenyl)imidazo[1,2-*a*]Quinoxalin-4-amin, *N*-Methyl-1-(3-Ethoxyphenyl)imidazo[1,2-*a*]Quinoxalin-4-amin, *N*-Methyl-1-(4-Ethoxyphenyl)imidazo[1,2-*a*]Quinoxalin-4-amin, *N*-Methyl-1-(3-Bromophenyl)imidazo[1,2-*a*]Quinoxalin-4-amin, *N-*Methyl-1-(4-Bromophenyl)imidazo[1,2-*a*]Quinoxalin-4-amin, *N*-Methyl-1-(3-(Trifluoromethyl)phenyl))imidazo[1,2-*a*]Quinoxalin-4-amin, *N*-Methyl-1-(4-(Trifluoromethyl)phenyl))imidazo[1,2-*a*]Quinoxalin-4-amin, *N*-Methyl-1-(3-Chlorophenyl)imidazo[1,2-*a*]Quinoxalin-4-amin, *N*-Methyl-1-(4-Chlorophenyl)imidazo[1,2-*a*]Quinoxalin-4-amin, *N*-Methyl-1-(3-Carboxyphenyl)imidazo[1,2-*a*]Quinoxalin-4-amin, *N-*Methyl-1-(4-Carboxyphenyl)imidazo[1,2-*a*]Quinoxalin-4-amin, *N*-Methyl-1-(3-Fluorphenyl)imidazo[1,2-*a*]Quinoxalin-4-amin, *N*-Methyl-1-(4-Fluorphenyl)imidazo[1,2-*a*]Quinoxalin-4-amin, *N*-Methyl-1-(3-Cyanophenyl)imidazo[1,2-*a*]Quinoxalin-4-amin, *N-*Methyl-1-(4-Cyanophenyl)imidazo[1,2-*a*]Quinoxalin-4-amin, *N*-Methyl-1-(3-Nitrophenyl)imidazo[1,2-*a*]Quinoxalin-4-amin, *N*-Methyl-1-(4-Nitrophenyl)imidazo[1,2-*a*]Quinoxalin-4-amin, *N*-(2-Aminoethyl)-1-(2-Hydroxyphenyl)imidazo[1,2-*a*]Quinoxalin-4-amin, *N*-(2-Aminoethyl)-1-(3-Hydroxyphenyl)imidazo[1,2-*a*]Quinoxalin-4-amin, *N*-(2-Aminoethyl)-1-(4-Hydroxyphenyl)imidazo[1,2-*a*]Quinoxalin-4-amin, *N*-(2-Aminoethyl)-1-(2,4-Dihydroxyphenyl)imidazo[1,2-*a*]Quinoxalin-4-amin, *N*-(2-Aminoethyl)-1-(2,3-Dihydroxyphenyl)imidazo[1,2-*a*]Quinoxalin-4-amin, *N*-(2-Aminoethyl)-1-(2-Methoxyphenyl)imidazo[1,2-*a*]Quinoxalin-4-amin, *N*-(2-Aminoethyl)-1-(3-Methoxyphenyl)imidazo[1,2-*a*]Quinoxalin-4-amin, *N*-(2-Aminoethyl)-1-(4-Methoxyphenyl)imidazo[1,2-*a*]Quinoxalin-4-amin, *N*-(2-Aminoethyl)-1-(2,4-Dimethoxyphenyl)imidazo[1,2-*a*]Quinoxalin-4-amin, *N*-(2-Aminoethyl)-1-(2,3-Dimethoxyphenyl)imidazo[1,2-*a*]Quinoxalin-4-amin, *N*-(2-Aminoethyl)-1-(3-Ethoxyphenyl)imidazo[1,2-*a*]Quinoxalin-4-amin, *N*-(2-Aminoethyl)-1-(4-Ethoxyphenyl)imidazo[1,2-*a*]Quinoxalin-4-amin, *N*-(2-Aminoethyl)-1-(3-Bromophenyl)imidazo[1,2-*a*]Quinoxalin-4-amin, *N*-(2-Aminoethyl)-1-(4-Bromophenyl)imidazo[1,2-*a*]Quinoxalin-4-amin, *N*-(2-Aminoethyl)-1-(3-(Trifluoromethyl)phenyl))imidazo[1,2-*a*]Quinoxalin-4-amin, *N*-(2-Aminoethyl)-1-(4-(Trifluoromethyl)phenyl))imidazo[1,2-*a*]Quinoxalin-4-amin, *N*-(2-Aminoethyl)-1-(3-Chlorophenyl)imidazo[1,2-*a*]Quinoxalin-4-amin, *N*-(2-Aminoethyl)-1-(4-Chlorophenyl)imidazo[1,2-*a*]Quinoxalin-4-amin, *N*-(2-Aminoethyl)-1-(3-Carboxyphenyl)imidazo[1,2-*a*]Quinoxalin-4-amin, *N*-(2-Aminoethyl)-1-(4-Carboxyphenyl)imidazo[1,2-*a*]Quinoxalin-4-amin, *N*-(2-Aminoethyl)-1-(3-Fluorphenyl)imidazo[1,2-*a*]Quinoxalin-4-amin, *N*-(2-Aminoethyl)-1-(4-Fluorphenyl)imidazo[1,2-*a*]Quinoxalin-4-amin, *N*-(2-Aminoethyl)-1-(3-Cyanophenyl)imidazo[1,2-*a*]Quinoxalin-4-amin, *N*-(2-Aminoethyl)-1-(4-Cyanophenyl)imidazo[1,2-*a*]Quinoxalin-4-amin, *N*-(2-Aminoethyl)-1-(3-Nitrophenyl)imidazo[1,2-*a*]Quinoxalin-4-amin, *N*-(2-Aminoethyl)-1-(4-Nitrophenyl)imidazo[1,2-*a*]Quinoxalin-4-amin, 1-Phenylimidazo[1,2-*a*]Quinoxalin-4-amin, 1-(2,4-Dihydroxyphenyl)imidazo[1,2-*a*]Quinoxalin-4-amin, 1-(2,3-Dihydroxyphenyl)imidazo[1,2-*a*]Quinoxalin-4-amin, 1-(2-Methoxyphenyl)imidazo[1,2-*a*]Quinoxalin-4-amin, 1-(3-Methoxyphenyl)imidazo[1,2-*a*]Quinoxalin-4-amin, 1-(4-Methoxyphenyl)imidazo[1,2-*a*]Quinoxalin-4-amin, 1-(2,4-Dimethoxyphenyl)imidazo[1,2-*a*]Quinoxalin-4-amin, 1-(2,3-Dimethoxyphenyl)imidazo[1,2-*a*]Quinoxalin-4-amin, 1-(3-Ethoxyphenyl)imidazo[1,2-*a*]Quinoxalin-4-amin, 1-(4-Ethoxyphenyl)imidazo[1,2-*a*]Quinoxalin-4-amin, 1-(3-Bromophenyl)imidazo[1,2-*a*]Quinoxalin-4-amin, 1-(4-Bromophenyl)imidazo[1,2-*a*]Quinoxalin-4-amin, 1-(3-(Trifluoromethyl)phenyl))imidazo[1,2-*a*]Quinoxalin-4-amin, 1-(4-(Trifluoromethyl)phenyl))imidazo[1,2-*a*]Quinoxalin-4-amin, 1-(3-Chlorophenyl)imidazo[1,2-*a*]Quinoxalin-4-amin, 1-(4-Chlorophenyl)imidazo[1,2-*a*]Quinoxalin-4-amin, 1-(3-Carboxyphenyl)imidazo[1,2-*a*]Quinoxalin-4-amin, 1-(4-Carboxyphenyl)imidazo[1,2-*a*]Quinoxalin-4-amin, 1-(3-Fluorphenyl)imidazo[1,2-*a*]Quinoxalin-4-amin, 1-(4-Fluorphenyl)imidazo[1,2-*a*]Quinoxalin-4-amin, 1-(3-Cyanophenyl)imidazo[1,2-*a*]Quinoxalin-4-amin, 1-(4-Cyanophenyl)imidazo[1,2-*a*]Quinoxalin-4-amin, 1-(3-Nitrophenyl)imidazo[1,2-*a*]Quinoxalin-4-amin, 1-(4-Nitrophenyl)imidazo[1,2-*a*]Quinoxalin-4-amin, 4-Chloro-1-Phenylimidazol[1,2-*a*]Quinoxalin, 4-Chloro-1-(2-Hydroxyphenyl)imidazo[1,2-*a*]Quinoxalin, 4-Chloro-1-(3-Hydroxyphenyl)imidazo[1,2-*a*]Quinoxalin, 4-Chloro-1-(4-Hydroxyphenyl)imidazo[1,2-*a*]Quinoxalin, 4-Chloro-1-(2-Methoxyphenyl)imidazo[1,2-*a*]Quinoxalin, 4-Chloro-1-(3-Methoxyphenyl)imidazo[1,2-*a*]Quinoxalin, 4-Chloro-1-(4-Methoxyphenyl)imidazo[1,2-*a*]Quinoxalin, 4-Chloro-1-(2,4-Dimethoxyphenyl)imidazo[1,2-*a*]Quinoxalin, 4-Chloro-1-(2,3-Dimethoxyphenyl)imidazo[1,2-*a*]Quinoxalin, 4-Methoxy-1-Phenylimidazol[1,2-*a*]Quinoxalin, 4-Methoxy-1-(2-Hydroxyphenyl)imidazo[1,2-*a*]Quinoxalin, 4-Methoxy-1-(3-Hydroxyphenyl)imidazo[1,2-*a*]Quinoxalin, 4-Methoxy-1-(4-Hydroxyphenyl)imidazo[1,2-*a*]Quinoxalin, 4-Methoxy-1-(2-Methoxyphenyl)imidazo[1,2-*a*]Quinoxalin, 4-Methoxy-1-(3-Methoxyphenyl)imidazo[1,2-*a*]Quinoxalin, 4-Methoxy-1-(4-Methoxyphenyl)imidazo[1,2-*a*]Quinoxalin, 4-Methoxy-1-(2,4-Dimethoxyphenyl)imidazo[1,2-*a*]Quinoxalin, 4-Methoxy-1-(2,3-Dimethoxyphenyl)imidazo[1,2-*a*]Quinoxalin und ihre physiologisch annehmbaren Salze.

7. Verbindung zur Behandlung von Krebserkrankungen nach Anspruch 1, die unter den folgenden Verbindungen gewählt ist:
*N*-Methyl-1-(3-Hydroxyphenyl)imidazo[1,2-*a*]Quinoxalin-4-amin, *N*-Methyl-1-(3-Methoxyphenyl)imidazo[1,2-*a*]Quinoxalin-4-amin, 1-(3-Methoxyphenyl)imidazo[1,2-*a*]Quinoxalin-4-amin und ihre physiologisch annehmbaren Salze.

8. Verbindung zur Behandlung von Krebserkrankungen nach einem beliebigen der vorstehenden Ansprüche, **dadurch gekennzeichnet, dass** der Krebs aus Melanomen und Lymphomen ausgewählt ist.

9. Pharmazeutische Zusammensetzung zur Behandlung von Krebserkrankungen, umfassend eine Verbindung nach einem beliebigen der vorstehenden Ansprüche und einen geeigneten pharmazeutischen Träger.

10. Produkt, umfassend eine Verbindung zur Behandlung von Krebserkrankungen nach einem beliebigen der vorstehenden Ansprüche 1 bis 7 und einen anderen Wirkstoff als Kombinationsprodukt zur gleichzeitigen, getrennten oder gestaffelten Verwendung in der Therapie.

## Claims

1. A compound for the treatment of cancers of formula (IX): 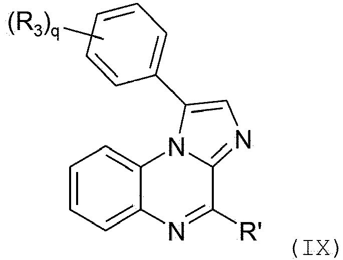 wherein:
R₃ represents a hydrogen atom, a halogen or a group selected among the groups methyl, ethyl, propyl, isopropyl, butyl, isobutyl, tertiobutyl, -CF₃, CN, CHO, NO₂ and - (CH₂)_{n"}-(CH=CH)-(CH₂)_{n'"}-CH₃ with n" and n'" independently between 0 and 4, COOR₄, NR₄R₅ and OR₄,
R₄ and R₅ represent independently a hydrogen atom or a group selected among the linear or branched C₁-C₄ alkyl, linear or branched C₁-C₄ alkenyl, C₃-C₇ cycloalkyl radicals,
q is between 1 and 5,
R' represents a chloro, methoxy, amino, methylamino, dimethylamino, ethylamino, diethylamino, aminomethylamine or aminoethylamine group,
and physiologically acceptable salts of same.

2. The compound for the treatment of cancers according to claim 1 wherein R₃ is selected among H, Cl, Br, F, hydroxy, methyl, methoxy, ethoxy, -CF₃, CN, COOH, COOCH₃, COOCH₂CH₃.

3. The compound for the treatment of cancers according to one of the preceding claims wherein R₃ is selected among H, hydroxy, methoxy, ethoxy, Br, CF₃, Cl and COOH.

4. The compound for the treatment of cancers according to one of the preceding claims wherein R' is -NH-CH₃, -NH₂ or -NH-(CH₂)₂-NH₂.

5. The compound for the treatment of cancers according to one of the preceding claims wherein R' is -NH-CH₃ or -NH₂; R₃ is hydroxy or methoxy and q = 1.

6. The compound for the treatment of cancers according to claim 1 selected among the following compounds:
*N*-methyl-1-phenylimidazo[1,2-*a*]quinoxalin-4-amine, *N-*methyl-1-(2-hydroxyphenyl)imidazo[1,2-*a*]quinoxalin-4-amine, *N*-methyl-1-(3-hydroxyphenyl)imidazo[1,2-*a*]quinoxalin-4-amine, *N*-methyl-1-(4-hydroxyphenyl)imidazo[1,2-*a*]quinoxalin-4-amine, *N-*methyl-1-(2,4-dihydroxyphenyl)imidazo[1,2-*a*]quinoxalin-4-amine, *N*-methyl-1-(2,3-dihydroxyphenyl)imidazo[1,2-*a*]quinoxalin-4-amine, *N*-methyl-1-(2-methoxyphenyl)imidazo[1,2-*a*]quinoxalin-4-amine, *N-*methyl-1-(3-methoxyphenyl)imidazo[1,2-*a*]quinoxalin-4-amine, *N*-methyl-1-(4-methoxyphenyl)imidazo[1,2-*a*]quinoxalin-4-amine, *N*-methyl-1-(2,4-dimethoxyphenyl)imidazo[1,2-*a*]quinoxalin-4-amine, *N-*methyl-1-(2,3-dimethoxyphenyl)imidazo[1,2-*a*]quinoxalin-4-amine, *N*-methyl-1-(3-ethoxyphenyl)imidazo[1,2-*a*]quinoxalin-4-amine, *N*-methyl-1-(4-ethoxyphenyl)imidazo[1,2-*a*]quinoxalin-4-amine, *N-*methyl-1-(3-bromophenyl)imidazo[1,2-*a*]quinoxalin-4-amine, *N*-methyl-1-(4-bromophenyl)imidazo[1,2-*a*]quinoxalin-4-amine, *N*-methyl-1-(3-(trifluoromethyl)phenyl))imidazo[1,2-*a*]quinoxalin-4-amine, *N*-methyl-1-(4-(trifluoromethyl)phenyl))imidazo[1,2-*a*]quinoxalin-4-amine, *N*-methyl-1-(3-chlorophenyl)imidazo[1,2-*a*]quinoxalin-4-amine, *N*-methyl-1-(4-chlorophenyl)imidazo[1,2-*a*]quinoxalin-4-amine, *N-*methyl-1-(3-carboxyphenyl)imidazo[1,2-*a*]quinoxalin-4-amine, *N*-methyl-1-(4-carboxyphenyl)imidazo[1,2-*a*]quinoxalin-4-amine, *N*-methyl-1-(3-fluorophenyl)imidazo[1,2-a]quinoxalin-4-amine, *N-*methyl-1-(4-fluorophenyl)imidazo[1,2-*a*]quinoxalin-4-amine, *N*-methyl-1-(3-cyanophenyl)imidazo[1,2-*a*]quinoxalin-4-amine, *N*-methyl-1-(4-cyanophenyl)imidazo[1,2-*a*]quinoxalin-4-amine, *N*-methyl-1-(3-nitrophenyl)imidazo[1,2-*a*]quinoxalin-4-amine, *N-*methyl-1-(4-nitrophenyl)imidazo[1,2-*a*]quinoxalin-4-amine, *N*-(2-aminoethyl)-1-(2-hydroxyphenyl)imidazo[1,2-*a*]quinoxalin-4-amine, *N*-(2-aminoethyl)-1-(3-hydroxyphenyl)imidazo[1,2-*a*]quinoxalin-4-amine, *N*-(2-aminoethyl)-1-(4-hydroxyphenyl)imidazo[1,2-*a*]quinoxalin-4-amine, *N*-(2-aminoethyl)-1-(2,4-dihydroxyphenyl)imidazo[1,2-*a*]quinoxalin-4-amine, *N*-(2-aminoethyl)-1-(2,3-dihydroxyphenyl)imidazo[1,2-*a*]quinoxalin-4-amine, *N*-(2-aminoethyl)-1-(2-methoxyphenyl)imidazo[1,2-*a*]quinoxalin-4-amine, *N*-(2-aminoethyl)-1-(3-methoxyphenyl)imidazo[1,2-*a*]quinoxalin-4-amine, *N*-(2-aminoethyl)-1-(4-methoxyphenyl)imidazo[1,2-*a*]quinoxalin-4-amine, *N*-(2-aminoethyl)-1-(2,4-dimethoxyphenyl)imidazo[1,2-*a*]quinoxalin-4-amine, *N*-(2-aminoethyl)-1-(2,3-dimethoxyphenyl)imidazo[1,2-*a*]quinoxalin-4-amine, *N*-(2-aminoethyl)-1-(3-ethoxyphenyl)imidazo[1,2-*a*]quinoxalin-4-amine, *N*-(2-aminoethyl)-1-(4-ethoxyphenyl)imidazo[1,2-*a*]quinoxalin-4-amine, *N*-(2-aminoethyl)-1-(3-bromophenyl)imidazo[1,2-*a*]quinoxalin-4-amine, *N*-(2-aminoethyl)-1-(4-bromophenyl)imidazo[1,2-*a*]quinoxalin-4-amine, *N*-(2-aminoethyl)-1-(3-(trifluoromethyl)phenyl))imidazo[1,2-*a*]quinoxalin-4-amine, *N*-(2-aminoethyl)-1-(4-(trifluoromethyl)phenyl))imidazo[1,2-*a*]quinoxalin-4-amine, *N*-(2-aminoethyl)-1-(3-chlorophenyl)imidazo[1,2-*a*]quinoxalin-4-amine, *N*-(2-aminoethyl)-1-(4-chlorophenyl)imidazo[1,2-*a*]quinoxalin-4-amine, *N*-(2-aminoethyl)-1-(3-carboxyphenyl)imidazo[1,2-*a*]quinoxalin-4-amine, *N*-(2-aminoethyl)-1-(4-carboxyphenyl)imidazo[1,2-*a*]quinoxalin-4-amine, *N*-(2-aminoethyl)-1-(3-fluorophenyl)imidazo[1,2-*a*]quinoxalin-4-amine, *N*-(2-aminoethyl)-1-(4-fluorophenyl)imidazo[1,2-*a*]quinoxalin-4-amine, *N*-(2-aminoethyl)-1-(3-cyanophenyl)imidazo[1,2-*a*]quinoxalin-4-amine, *N*-(2-aminoethyl)-1-(4-cyanophenyl)imidazo[1,2-*a*]quinoxalin-4-amine, *N*-(2-aminoethyl)-1-(3-nitrophenyl)imidazo[1,2-*a*]quinoxalin-4-amine, *N*-(2-aminoethyl)-1-(4-nitrophenyl)imidazo[1,2-*a*]quinoxalin-4-amine, 1-phenylimidazo[1,2-*a*]quinoxalin-4-amine, 1-(2,4-dihydroxyphenyl)imidazo[1,2-*a*]quinoxalin-4-amine, 1-(2,3-dihydroxyphenyl)imidazo[1,2-*a*]quinoxalin-4-amine, 1-(2-methoxyphenyl)imidazo[1,2-*a*]quinoxalin-4-amine, 1-(3-methoxyphenyl)imidazo[1,2-*a*]quinoxalin-4-amine, 1-(4-methoxyphenyl)imidazo[1,2-*a*]quinoxalin-4-amine, 1-(2,4-dimethoxyphenyl)imidazo[1,2-*a*]quinoxalin-4-amine, 1-(2,3-dimethoxyphenyl)imidazo[1,2-*a*]quinoxalin-4-amine, 1-(3-ethoxyphenyl)imidazo[1,2-*a*]quinoxalin-4-amine, 1-(4-ethoxyphenyl)imidazo[1,2-*a*]quinoxalin-4-amine, 1-(3-bromophenyl)imidazo[1,2-*a*]quinoxalin-4-amine, 1-(4-bromophenyl)imidazo[1,2-*a*]quinoxalin-4-amine, 1-(3-(trifluoromethyl)phenyl))imidazo[1,2-*a*]quinoxalin-4-amine, 1-(4-(trifluoromethyl)phenyl))imidazo[1,2-*a*]quinoxalin-4-amine, 1-(3-chlorophenyl)imidazo[1,2-*a*]quinoxalin-4-amine, 1-(4-chlorophenyl)imidazo[1,2-*a*]quinoxalin-4-amine, 1-(3-carboxyphenyl)imidazo[1,2-*a*]quinoxalin-4-amine, 1-(4-carboxyphenyl)imidazo[1,2-*a*]quinoxalin-4-amine, 1-(3-fluorophenyl)imidazo[1,2-*a*]quinoxalin-4-amine, 1-(4-fluorophenyl)imidazo[1,2-*a*]quinoxalin-4-amine, 1-(3-cyanophenyl)imidazo[1,2-*a*]quinoxalin-4-amine, 1-(4-cyanophenyl)imidazo[1,2-*a*]quinoxalin-4-amine, 1-(3-nitrophenyl)imidazo[1,2-*a*]quinoxalin-4-amine, 1-(4-nitrophenyl)imidazo[1,2-*a*]quinoxalin-4-amine, 4-chloro-1-phenylimidazo[1,2-*a*]quinoxaline, 4-chloro-1-(2-hydroxyphenyl)imidazo[1,2-*a*]quinoxaline, 4-chloro-1-(3-hydroxyphenyl)imidazo[1,2-*a*]quinoxaline, 4-chloro-1-(4-hydroxyphenyl)imidazo[1,2-*a*]quinoxaline, 4-chloro-1-(2-methoxyphenyl)imidazo[1,2-*a*]quinoxaline, 4-chloro-1-(3-methoxyphenyl)imidazo[1,2-*a*]quinoxaline, 4-chloro-1-(4-methoxyphenyl)imidazo[1,2-*a*]quinoxaline, 4-chloro-1-(2,4-dimethoxyphenyl)imidazo[1,2-*a*]quinoxaline, 4-chloro-1-(2,3-dimethoxyphenyl)imidazo[1,2-*a*]quinoxaline, 4-methoxy-1-phenylimidazo[1,2-*a*]quinoxaline, 4-methoxy-1-(2-hydroxyphenyl)imidazo[1,2-*a*]quinoxaline, 4-methoxy-1-(3-hydroxyphenyl)imidazo[1,2-*a*]quinoxaline, 4-methoxy-1-(4-hydroxyphenyl)imidazo[1,2-*a*]quinoxaline, 4-methoxy-1-(2-methoxyphenyl)imidazo[1,2-*a*]quinoxaline, 4-methoxy-1-(3-methoxyphenyl)imidazo[1,2-*a*]quinoxaline, 4-methoxy-1-(4-methoxyphenyl)imidazo[1,2-*a*]quinoxaline, 4-methoxy-1-(2,4-dimethoxyphenyl)imidazo[1,2-*a*]quinoxaline, 4-methoxy-1-(2,3-dimethoxyphenyl)imidazo[1,2-*a*]quinoxaline, and physiologically acceptable salts of same.

7. The compound for the treatment of cancers according to claim 1 selected among the following compounds:
*N*-methyl-1-(3-hydroxyphenyl)imidazo[1,2-*a*]quinoxalin-4-amine, *N*-methyl-1-(3-methoxyphenyl)imidazo[1,2-*a*]quinoxalin-4-amine, 1-(3-methoxyphenyl)imidazo[1,2-*a*]quinoxalin-4-amine and physiologically acceptable salts of same.

8. The compound for the treatment of cancers according to one of the preceding claims **characterised in that** the cancer is selected among melanomas and lymphomas.

9. A pharmaceutical composition for the treatment of cancers comprising a compound according to one of the preceding claims and a suitable pharmaceutical carrier.

10. A product comprising a compound for the treatment of cancers according to one of claims 1 to 7 and another active agent as a combination product for simultaneous, separated or extended use over time in therapy.
